(19) Europäisches Patentamt
European Patent Office
Office européen des brevets

(11) EP 2 335 677 A1

(12) DEMANDE DE BREVET EUROPEEN

(43) Date de publication:
**22.06.2011 Bulletin 2011/25**

(21) Numéro de dépôt: **10194784.4**

(22) Date de dépôt: **13.12.2010**

(51) Int Cl.:
***A61K 8/34*** *(2006.01)* ***A61Q 1/10*** *(2006.01)*
***A61K 8/55*** *(2006.01)* ***A61K 8/92*** *(2006.01)*
***A45D 34/04*** *(2006.01)* ***A45D 40/26*** *(2006.01)*

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **17.12.2009 FR 0959140**
**17.12.2009 FR 0959147**

(71) Demandeur: **L'Oréal**
**75008 Paris (FR)**

(72) Inventeur: **Raineau, Olivier**
**75013, Paris (FR)**

(74) Mandataire: **Le Coupanec, Pascale A.M.P.**
**Nony & Associés**
**3, rue de Penthièvre**
**75008 Paris (FR)**

# (54) Composition cosmetique chargeante comprenant de l'alcool behenique a titre d'agent epaississant

(57) La présente invention concerne une composition cosmétique pour le maquillage et/ou le soin des fibres kératiniques, comprenant au moins un système émulsionnant exempt de stéarate de triéthanolamine, caractérisée en ce qu'elle contient de l'alcool béhénique, et en ce que la valeur de texture mesurée par texturométrie à compter de la préparation de ladite composition, à savoir à 24 heures après la fabrication de la composition, est supérieure à 20 g à température ambiante.

EP 2 335 677 A1

## Description

La présente invention concerne le domaine des compositions cosmétiques de maquillage et/ou de soin des fibres kératiniques, comprenant un système émulsionnant exempt de stéarate de triéthanolamine. Avantageusement, ces compositions possèdent des propriétés chargeantes

La texture de telles compositions, et en particulier la stabilité de la texture au cours du temps est déterminante pour les utilisateurs.

En effet, il a pu être observé dans le domaine des mascaras, et notamment des mascaras dits « chargeants », et plus particulièrement des mascaras comprenant de l'alcool cétylique à titre d'agent épaississant, que l'évolution de la texture de ces derniers est préjudiciable à la qualité du maquillage au fur et à mesure de leur utilisation.

Autrement dit, au cours de leur conservation et après ouverture de leur conditionnement, la composition « vieillit » et a tendance à voir sa texture augmenter au détriment des qualités d'application couramment recherchées, à savoir un dépôt aisé de produit sur les cils.

Cette évolution de la texture peut donner des mascaras adhérant moins et déposant moins.

Or on sait également que la matière déposée sur les fibres kératiniques ou charge, dépend outre de sa texture épaisse, de la dureté de l'applicateur qui peut notamment être une brosse. Plus précisément, plus un applicateur est souple, plus on parvient à déposer de matière. Toutefois, du fait de l'augmentation de la texture des compositions au cours du temps, on est souvent contraint d'utiliser des applicateurs comprenant des éléments d'application tels que des poils ou des dents plus durs afin de contrecarrer cette augmentation de texture. En effet, lorsque le couple applicateur/texture de la composition n'est pas adéquat, il arrive que l'on rencontre le défaut connu sous le nom de « brosse en sapin », où les poils de l'applicateur, en l'occurrence la brosse, se couchent lors du passage de l'essoreur et ne se redressent pas car ils n'ont pas une rigidité suffisante.

Par conséquent, avant une mise sur le marché, une étude est généralement réalisée sur l'adéquation entre la texture de la composition et la dureté de l'applicateur tant à compter de la préparation de la composition qu'une fois que cette dernière a vieilli. Du fait de la contrainte exposée ci-dessus, des applicateurs d'une dureté supérieure à celle théoriquement visée pour obtenir la charge souhaitée sont alors choisis, au détriment d'applicateurs plus souples, pourtant mieux adaptés sur le plan de l'application.

Ainsi, il existe un besoin de disposer de compositions cosmétiques, et notamment de mascaras chargeants, offrant une texture à l'issue de la fabrication relativement importante, qui ne doive pas être minimisée du fait de l'anticipation de l'évolution de la texture au cours du temps, évoquée ci-dessus, permettant d'employer des applicateurs plus souples que ceux couramment employés pour des compositions équivalentes non-conformes à la présente invention.

Les inventeurs ont mis en évidence les avantages liés à l'utilisation de l'alcool béhénique, notamment à titre d'agent épaississant, et notamment en remplacement total ou partiel de l'alcool cétylique couramment utilisé à ces mêmes fins, pour pallier les inconvénients précités.

La présente invention a ainsi pour objet une composition cosmétique pour le maquillage et/ou le soin des fibres kératiniques comprenant au moins un système émulsionnant exempt de stéarate de triéthanolamine, caractérisée en ce qu'elle contient de l'alcool béhénique, et en ce que la valeur de texture mesurée par texturométrie à compter de la préparation de ladite composition, à savoir 24 heures après fabrication de la composition, est supérieure à 20 g à température ambiante.

Plus particulièrement, la présente invention a pour objet une composition cosmétique pour le maquillage et/ou le soin des fibres kératiniques comprenant au moins un système émulsionnant exempt de stéarate de triéthanolamine, caractérisée en ce qu'elle contient au moins un pigment et de l'alcool béhénique, et en ce que la valeur de texture mesurée par texturométrie, selon la méthode de mesure de la texture indiquée dans la présente demande, à compter de la préparation de ladite composition, à savoir à 24 heures après la fabrication de la composition, est supérieure à 20 g à température ambiante, ledit alcool béhénique étant présent dans une teneur supérieure ou égale à 1 % en poids par rapport au poids total de la composition, ledit système émulsionnant comprenant au moins un agent tensioactif choisi parmi :

i) un alkyl phosphate de métal alcalin ou oxyde de phosphine de formule $(R\text{-}O)_n\text{-}P{=}O\text{-}(O^{-}M)m$ avec R représentant un groupe alkyle, linéaire ou ramifié, en $C_8$-$C_{22}$ tel que le cétyle, n étant égal à 1, 2 ou 3 et m étant égal à 0, 1 ou 2, avec m + n étant égal à 3 et M représentant un atome d'hydrogène ou un métal alcalin ou alcalino terreux, préférentiellement n = 1 et m = 2, et M est un métal alcalin, tel que le sodium ou le potassium,

ii) un alcool polyéthoxylé de formule R' $-(OCH_2CH_2)p\text{-}OH$ avec R' représentant un alkyle linéaire ou ramifié en $C_1$-$C_{30}$ et particulièrement représente $CH_3\text{-}(CH_2)_{17}\text{-}$ et p représentant un entier compris inclusivement entre 1 et 30, préférentiellement entre 2 et 20 ; tels que le stéareth-20 et le stéareth-2,

iii) un sel d'acide glutamique de formule R-CONH-C(COO-M)-$C_{2\text{-}4}$-COO-M' avec R représentant un groupe alkyle, linéaire ou ramifié en $C_8$-$C_{22}$ tel que le stéaryle et M' représentant un métal alcalin ou alcalino terreux, et

iv)un alkyl glucoside obtenu par condensation de glucose et d'alcools gras linéaires ou ramifiés en $C_8$-$C_{22}$ tel qu'un

mélange cétylique et stéarique nommé cétylstéaryle.

La méthode de mesure de texturométrie est avantageusement celle décrite dans la description ci-après.

La présente invention a également pour objet l'utilisation d'alcool béhénique, notamment à titre d'agent épaississant, dans une composition cosmétique de maquillage et/ou de soin des fibres kératiniques, et comprenant au moins un système émulsionnant exempt de stéarate de triéthanolamine, pour améliorer la stabilité de la texture de ladite composition.

La présente invention a encore pour objet un ensemble de conditionnement et d'application comprenant un récipient renfermant une composition telle que précédemment définie et un applicateur configuré pour appliquer ladite composition sur une matière kératiniques, et en particulier sur les fibres kératiniques, telles que les cils ou sourcils, ledit applicateur comprenant des éléments d'application, tels que des poils ou des dents, présentant une dureté comprise entre 20 Shore A et 40 Shore D.

Selon un autre aspect, la présente invention a également pour objet un procédé de revêtement, et notamment de maquillage et/ou de soin, des fibres kératiniques, telles que les cils ou les sourcils, comprenant une étape d'application d'une composition telle que précédemment définie sur lesdites fibres kératiniques.

Outre le besoin de mascaras chargeants, il existe également un besoin de disposer de compositions cosmétiques, et notamment de mascaras, offrant une texture stable dans le temps, autrement dit une variation de texture maîtrisée et limitée, assurant une application reproductible sur les fibres kératiniques.

Ainsi, selon un deuxième aspect, la présente invention a également pour objet une composition cosmétique pour le maquillage et/ou le soin des fibres kératiniques comprenant au moins un système émulsionnant exempt de stéarate de triéthanolamine, caractérisée en ce qu'elle contient de l'alcool béhénique, et en ce que la variation de texture mesurée par texturométrie dans une période de 60 jours à 45 °C à compter de la préparation de ladite composition, à savoir 24 heures après fabrication de la composition, est inférieure à 100 %, la variation de texture étant définie par :

$$\frac{T_{60\,j} - T_0}{T_0} \times 100$$

où $T_{60j}$ est la mesure de texturométrie à 60 jours, et

$T_0$ est la mesure de texturométrie faite 24 heures après fabrication de la préparation de la composition.

Toujours selon ce deuxième objet, la présente invention a pour objet l'utilisation d'alcool béhénique, notamment à titre d'agent épaississant, dans une composition cosmétique de maquillage et/ou de soin des fibres kératiniques, et comprenant au moins un système émulsionnant exempt de stéarate de triéthanolamine, pour améliorer la stabilité de la texture de ladite composition.

Toujours selon ce deuxième objet, la présente invention a encore pour objet un ensemble de conditionnement et d'application comprenant un récipient renfermant une composition telle que précédemment définie selon le deuxième aspect, et un applicateur configuré pour appliquer ladite composition sur une matière kératiniques, et en particulier sur les fibres kératiniques, telles que les cils ou sourcils, ledit applicateur comprenant des éléments d'application, tels que des poils ou des dents, présentant une dureté comprise entre 20 Shore A et 40 Shore D.

Toujours selon ce deuxième aspect, la présente invention a également pour objet un procédé de revêtement, et notamment de maquillage et/ou de soin des fibres kératiniques, telles que les cils ou les sourcils, comprenant une étape d'application d'une composition telle que précédemment définie selon le deuxième aspect sur lesdites fibres kératiniques.

Pour des raisons de simplification, on emploie dans la suite de la description la terminologie premier aspect de l'invention et deuxième aspect de l'invention pour désigner les deux modes de réalisation particuliers décrits ci-dessus.

Lorsque cela n'est pas précisé, la description se rapporte indifféremment aux deux aspects de l'invention.

## METHODE DE MESURE DE LA TEXTURE

La texture des mascaras est mesurée selon le protocole suivant :

L'appareil de mesure est un TA-XT2 vendu par la société Rhéo, équipé d'une cellule de mesure de force de 5 kgs et d'un mobile cylindrique de 12,7 mm (1/2 inch) de diamètre en Delrin. Le mascara est thermostaté à 20°C. Puis, il est placé en excès dans un contenant de diamètre 60 mm et de profondeur 22 mm à l'aide d'une spatule métallique. Le produit est étalé pour éviter toute poche d'air mais sans le triturer de façon à ne pas le déstructurer. On arase

ensuite le contenant à l'aide d'une spatule de façon à avoir la surface la plus régulière possible. Le contenant est ensuite couvert d'un verre de montre de façon à limiter l'évaporation de solvants présents dans la formule.

Les options retenues pour cette méthode de mesure sont les suivantes :

Mode de test : Mesure en compression
Force de déclenchement (trigger force): 2,0 g
Pré-vitesse : 2,0 mm/s
Vitesse de test 1,0 mm/s
Température 20 °C +/- 1 °C
Distance de pénétration 5 mm

On effectue trois mesures successives en des points séparés de 12 mm au minimum, à au moins 10 mm du bord du contenant. Le contenant est maintenu durant la mesure.
La valeur retenue est la moyenne des maxima obtenus à chaque mesure.
Selon un mode de réalisation particulier du premier aspect de l'invention, la texture, mesurée par texturométrie, à compter de la préparation de la composition, à savoir à $T_0$, est supérieure ou égale à 30g, voire à 60 g ou encore à 70 g à température ambiante.
Toujours selon un mode de réalisation du premier aspect de l'invention, la texture, mesurée par texturométrie, à l'issue d'une période de 60 jours à 45°C est inférieure ou égale à 100 g, voire à 90 g.
Toujours selon un autre mode de réalisation particulier du premier aspect de l'invention, la variation de texture mesurée par texturométrie dans une période de 60 jours à 45°C à compter de la préparation de ladite composition, à savoir 24 heures après fabrication de la composition, est inférieure à 100 %, la variation de texture étant définie par :

$$\frac{T_{60\,j} - T_0}{T_0} \times 100$$

où $T_{60j}$ est la mesure de texturamétrie à 60 jours, et
$T_0$ est la mesure de texturométrie faite 24 heures après fabrication de la préparation de la composition.
Selon un mode de réalisation particulier du deuxième aspect, la variation de texture mesurée par texturométrie dans une période de 60 jours à 45 °C à compter de la préparation de ladite composition est inférieure à 70 %, voire à 60 %, voire à 50 % par exemple à 20 %, 10 % ou encore 5 %.
Ainsi, selon son deuxième aspect, la présente invention permet de préparer des compositions cosmétiques présentant une texture plus importante que celle des compositions équivalentes actuellement commercialisées. Ainsi, l'augmentation de la texture au cours du temps étant moindre dans les compositions selon la présente invention que dans les compositions couramment préparées, la présente invention présente l'avantage de permettre de préparer des compositions cosmétiques d'une texture plus grande à l'issue de la fabrication. Autrement dit, il n'est plus nécessaire d'anticiper autant qu'habituellement l'évolution de texture des compositions cosmétiques considérées au cours du temps.

## ALCOOL BEHENIQUE

L'alcool béhénique, autrement nommé docosanol est un alcool gras en $C_{22}$.
L'alcool cétylique est couramment utilisé pour épaissir les compositions cosmétiques de type émulsion dans lesquelles le système tensioactif est exempt de stéarate de triéthanolamine, en particulier pour des compositions de mascaras dits « chargeants ». Il s'agit d'un co-tensioactif de bas HLB, couramment employé en association avec un tensioactif de haut HLB tel que le cétyl phosphate de potassium et/ou le stéareth-20.
Il a été observé que des compositions cosmétiques de type émulsion comprenant un tel système tensioactif ont tendance à voir leur texture évoluer au cours du temps, et plus précisément augmenter de manière gênante sur le plan de l'applicabilité de la composition sur les zones à maquiller et/ou à traiter.
Le remplacement total ou partiel de l'alcool cétylique dans ce type de compositions cosmétiques est précisément visé dans le cadre de la présente invention.
L'alcool béhénique peut être présent dans une teneur supérieure ou égale à 0,3 % en poids, en particulier à 0,5 % en poids, et plus particulièrement à 1 % en poids, voire à 2 % en poids par rapport au poids total de la composition.
Typiquement, l'alcool béhénique est présent dans une teneur variant de 0,3 % à 20 % en poids, notamment

de 0,5 à 10 % en poids, plus particulièrement de 0,7 % à 7 %, voire de 1 % à 6 % en poids par rapport au poids total de la composition.

**SYSTEME EMULSIONNANT**

La composition selon l'invention est exempte de stéarate de triéthanolamine. Autrement dit, elle contient moins de 1 % en poids de stéarate de triéthanolamine, mieux moins de 0,1 % en poids, voire 0 % en poids, par rapport au poids total de la composition.

Selon l'invention, on utilise généralement un système émulsionnant choisi de manière appropriée pour l'obtention d'une émulsion cire-dans-eau ou huile-dans-eau. En particulier, le système émulsionnant peut comprendre au moins un agent émulsionnant possédant à 25 °C une balance HLB (hydrophile-lipophile balance) au sens de GRIFFIN, supérieure ou égale à 8.

La valeur HLB selon GRIFFIN est définie dans J. Soc. Cosm. Chem. 1954 (volume 5), pages 249-256.

Ces agents émulsionnant peuvent être choisis parmi des tensioactifs non ioniques, anioniques, cationiques, amphotériques ou encore des tensioactifs polymériques. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier pages 347-377 de cette référence, pour les tensioactifs anioniques, amphotériques et non ioniques.

Les tensioactifs pouvant être utilisés dans la composition selon l'invention sont choisis parmi :

a) les tensioactifs non ioniques de HLB supérieur ou égal à 8 à 25°C, utilisés seuls ou en mélange. On peut citer notamment :

- les esters et éthers d'oses tels que le mélange de cétylstéaryl glucoside et d'alcools cétylique et stéarylique comme le Montanov 68 de Seppic;
- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool cétéarylique à 30 groupes oxyéthylénés (nom CTFA « Ceteareth-30 »), l'éther oxyéthyléné de l'alcool stéarylique à 20 groupes oxyéthylénés (nom CTFA « Stéareth-20 »), l'éther oxyéthyléné du mélange d'alcools gras en $C_{12}$-$C_{15}$ comportant 7 groupes oxyéthylénés (nom CTFA « $C_{12-15}$ Pareth-7 ») notamment commercialisé sous la dénomination NEODOL 25-7® par SHELL CHEMICALS
- les esters d'acide gras (notamment d'acide en $C_8$-$C_{24}$, et de préférence en $C_{16}$-$C_{22}$) et de polyéthylène glycol (pouvant comprendre de 1 à 150 motifs d'éthylèneglycol) tels que le stéarate de PEG-50 et le monostéarate de PEG-40 notamment, commercialisé sous le nom MYRJ 52P® par la société ICI UNIQUEMA, ou encore le PEG-30 glyceryl stéarate notamment commercialisé sous le nom TAGAT S® par la société Evonik GOLDSCHMIDT ;
- les esters d'acide gras (notamment d'acide en $C_8$-$C_{24}$, et de préférence en $C_{16}$-$C_{22}$) et des éthers de glycérol oxyéthylénés et/ou oxypropylénés (pouvant comporter de 1 à 150 groupes oxyéthylénés et/ou oxypropylénés), comme le monostéarate de PEG-200 glycéryle notamment vendu sous la dénomination Simulsol 220 TM® par la société SEPPIC ; le stéarate de glycéryle polyéthoxylé à 30 groupes d'oxyde d'éthylène comme le produit TAGAT S® vendu par la société Evonik GOLDSCHMIDT, l'oléate de glycéryle polyéthoxylé à 30 groupes d'oxyde d'éthylène comme le produit TAGAT O® vendu par la société Evonik GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société Evonik GOLDSCHMIDT et le laurate de glycéryle polyéthoxylé à 30 groupes d'oxyde d'éthylène comme le produit TAGAT I® de la société Evonik GOLDSCHMIDT,
- les esters d'acide gras (notamment d'acide en $C_8$-$C_{24}$, et de préférence en $C_{16}$-$C_{22}$) et des éthers de sorbitol oxyéthylénés et/ou oxypropylénés (pouvant comporter de 1 à 150 groupes oxyéthylénés et/ou oxypropylénés), comme le polysorbate 20 notamment vendu sous la dénomination Tween 20® par la société CRODA, le polysorbate 60 notamment vendu sous la dénomination Tween 60® par la société CRODA,
- la diméthicone copolyol, telle que celle vendue sous la dénomination Q2-5220® par la société DOW CORNING,
- la diméthicone copolyol benzoate (FINSOLV SLB 101® et 201® de la société FINTEX),
- les copolymères d'oxyde de propylène et d'oxyde d'éthylène, également appelés polycondensats OE/OP,
- et leurs mélanges.

Les polycondensats OE/OP sont plus particulièrement des copolymères consistant en des blocs polyéthylène glycol

et polypropylène glycol, comme par exemple les polycondensats tribloc polyéthylène glycol/polypropylène glycol/ polyéthylène glycol, Ces polycondensats tribloc ont par exemple la structure chimique suivante :

$$H-(O-CH_2CH_2)_a-(O-CH(CH_3)-CH_2)_b-(O-CH_2-CH_2)_a-OH,$$

formule dans laquelle a va de 2 à 120, et b va de 1 à 100.

Le polycondensat OE/OP a de préférence un poids moléculaire moyen en poids allant de 1000 à 15000, et mieux allant de 2000 à 13000. Avantageusement, ledit polycondensat OE/OP a une température de trouble, à 10 g/l en eau distillée, supérieure ou égale à 20°C, de préférence supérieure ou égale à 60°C. La température de trouble est mesurée selon la norme ISO 1065. Comme polycondensat OE/OP utilisable selon l'invention, on peut citer les polycondensats tribloc polyéthylène glycol/polypropylène glycol/polyéthylène glycol vendus sous les dénominations SYNPERONIC® comme les SYNPERONIC PE/ L44® et SYNPERONIC PE/F127® par la société ICI.

b) les tensioactifs non ioniques de HLB inférieur à 8 à 25°C, éventuellement associés à un ou plusieurs agents tensioactif non ioniques de HLB supérieur à 8 à 25°C tels que cités ci-dessus ; on peut citer notamment :

- les esters et éthers d'oses tels que le stéarate de sucrose, le cocoate de sucrose, le stéarate de sorbitan et leurs mélanges comme l'Arlatone 2121®commercialisé par la société ICI ;
- les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool stéarylique à 2 groupes oxyéthylénés (nom CTFA « Stéareth-2 ») ;
- les esters d'acides gras (notamment d'acide en $C_8$-$C_{24}$, et de préférence en $C_{16}C_{22}$) et de polyol, notamment de glycérol ou de sorbitol, tels que le stéarate de glycéryle, tel que le produit vendu sous la dénomination TEGIN M® par la société Evonik GOLDSCHMIDT, le laurate de glycéryle tel que le produit vendu sous la dénomination IMWITOR 312® par la société HULS, le stéarate de polyglycéryl-2, le tristéarate de sorbitan, le ricinoléate de glycéryle ;
- les lécithines, telles que les lécithines de soja (comme Emulmetik 100 J de Cargill, ou Biophilic H de Lucas Meyer) ;
- le mélange de cyclométhicone/diméthicone copolyol vendu sous la dénomination Q2-3225C® par la société DOW CORNING.

c) les tensioactifs anioniques tels que :

- les sels d'acides gras polyoxyéthylénés notamment ceux dérivant des amines ou les sels alcalins, et leurs mélanges ;
- les esters phosphoriques et leurs sels tels que le « DEA oleth-10 phosphate » (Crodafos N 10N de la société CRODA) ou le phosphate de monocétyle monopotassique ou cétyl phosphate de potassium (Amphisol K de Givaudan) ;
- les sulfosuccinates tels que le *« Disodium PEG-5 citrate lauryl sulfosuccinate* » et le *« Disodium ricinoleamido MEA sulfosuccinate* » ;
- les alkyléthersulfates tels que le lauryl éther sulfate de sodium ;
- les iséthionates ;
- les acylglutamates tels que le *« Disodium hydrogenated tallow glutamate »* (AMISOFT HS-21 R® commercialisé par la société AJINOMOTO) et le sodium stearoyl glutamate (AMISOFT HS-11 PF® commercialisé par la société AJINOMOTO) et leurs mélanges ;
- les dérivés de soja comme le potassium soyate ;
- les citrates, comme le Glyceryl stearate citrate (Axol C 62 Pellets de Degussa) ;
- les dérivés de proline, comme le Sodium palmitoyl proline (Sepicalm VG de Seppic), ou le Mélange de Sodium palmitoyle sarcosinate, Magnesium palmitoyle glutamate, acide palmitique et Palmitoyle proline (Sepifeel One de Seppic) ;
- les lactylates, comme le Sodium stearoyl lactylate (Akoline SL de Karlshamns AB) ;
- les sarcosinates, comme le sodium palmitoyle sarcosinate (Nikkol sarcosinate PN) ou le mélange de Stéaroyle sarcosine et Myristoyle sarcosine 75/25 (Crodasin SM de Croda) ;
- les sulfonates, comme le Sodium $C_{14}$-$_{17}$ alkyl sec sulfonate (Hostapur SAS 60 de Clariant) ;
- les glycinates, comme le sodium cocoyle glycinate (Amilite GCS-12 d'Ajinomoto).

Les compositions conformes à l'invention peuvent également contenir un ou plusieurs tensioactifs amphotériques comme les bétaines ou N-acyl-aminoacides tels que les N-alkyl-aminoacétates et le cocoamphodiacétate disodique et les oxydes d'amines tels que l'oxyde de stéaramine ou encore des tensioactifs siliconés comme les diméthicone

copolyols phosphates tels que celui vendu sous la dénomination PECOSIL PS 100® par la société PHOENIX CHEMICAL.

L'agent émulsionnant utilisable peut également être un tensioactif polymérique, notamment un polymère thermogélifiant.

Selon un mode de réalisation particulier, le système émulsionnant comprend au moins un agent tensioactif choisi parmi :

i) un alkyl phosphate de métal alcalin ou oxyde de phosphine de formule (R-O),-P=O-(O-M)$_m$ avec R représentant un groupe alkyle, linéaire ou ramifié, en $C_8$-$C_{22}$ tel que le cétyle, n étant égal à 1, 2 ou 3 et m étant égal à 0, 1 ou 2, avec m + n étant égal à 3 et M représentant un atome d'hydrogène ou un métal alcalin ou alcalino terreux, préférentiellement n = 1 et m = 2, et M est un métal alcalin, tel que le sodium ou le potassium,

ii) un alcool polyéthoxylé de formule R'-$(OCH_2CH_2)_p$-OH avec R' représentant un alkyle linéaire ou ramifié en $C_1$-$C_{30}$ et particulièrement représente $CH_3$-$(CH_2)_{17}$- et p représentant un entier compris inclusivement entre 1 et 30, préférentiellement entre 2 et 20 ; tels que le stéareth-20 et le stéareth-2,

iii) un sel d'acide glutamique de formule R-CONH-C($COO^-$M)-$C_2H_4$-COO-M' avec R représentant un groupe alkyle, linéaire ou ramifié en $C_8$-$C_{22}$ tel que le stéaryle et M' représentant un métal alcalin ou alcalino terreux, et

iv) un alkyl glucoside obtenu par condensation de glucose et d'alcools gras linéaires ou ramifiés en $C_8$-$C_{22}$ tel qu'un mélange cétylique et stéarique nommé cétylstéaryle.

A titre d'exemple de tensioactif selon le point (i) ci-dessus, on peut citer le cetyl phosphate de potassium notamment vendu sous le nom Amphisol K par la société Givaudan.

A titre d'exemple de tensioactif selon le point (iii) ci-dessus on peut citer le glutamate de stéaroyle de sodium et à titre de tensioactif selon le point (iv) ci-dessus on peut citer le glucoside de cétylstéaryle.

Selon un mode de réalisation encore plus particulier, le système émulsionnant comprend au moins un de ces deux agents tensioactifs ou leur mélange.

Selon un autre mode de réalisation particulier de l'invention, le système émulsionnant comprend au moins un agent émulsionnant choisi parmi (a) les éthers oxyéthylénés et/ou oxypropylénés (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tels que l'éther oxyéthyléné de l'alcool stéarylique à 2 groupes oxyéthylénés (nom CTFA « Stéareth-2 ») ; (b) les esters d'acides gras (notamment d'acide en $C_8$-$C_{24}$, et de préférence en $C_{16}$-$_{22}$ et de polyol, notamment de glycérol ou de sorbitol, tels que le stéarate de glycéryle tel que le produit vendu sous la dénomination TEGIN M® par la société Evonik GOLDSCHMIDT; (c) les esters d'acide phosphorique et leurs sels de métal alcalin tels que le cétyl phosphate de potassium (Amphisol K de Givaudan) et/ou (d) les esters d'acide gras (notamment d'acide en $C_8$-$C_{22}$, et de préférence en $C_{16}$-$C_{22}$) et des éthers de glycérol oxyéthylénés et/ou oxypropylénés (pouvant comporter de 1 à 150 groupes oxyéthylénés et/ou oxypropylénés), comme le stéarate de glycéryle polyéthoxylé à 30 groupes d'oxyde d'éthylène comme le produit TAGAT® S vendu par la société Evonik GOLDSCHMIDT et (e) leurs mélanges.

Selon un mode de réalisation particulier de l'invention, le système émulsionnant comprend au moins un éther oxyéthyléné et/ou oxypropyléné (pouvant comporter de 1 à 150 groupes oxyéthylénés et/ou oxypropylénés) d'alcools gras (notamment d'alcool en $C_8$-$C_{24}$, et de préférence en $C_{12}$-$C_{18}$) tel que l'éther oxyéthyléné de l'alcool stéarylique à 2 groupes oxyéthylénés (nom CTFA « Stéareth-2 »), et au moins un alkyl phosphate de métal alcalin ou oxyde de phosphine de formule $(R-O)_n$-P=O-$(O^-M)_m$ avec R représentant un groupe alkyle, linéaire ou ramifié, en $C_8$-$C_{22}$ tel que le cétyle, n étant égal à 1, 2 ou 3 et m étant égal à 0, 1 ou 2, avec m + n étant égal à 3 et M représentant un atome d'hydrogène ou un métal alcalin ou alcalino terreux, préférentiellement n = 1 et m = 2, et M est un métal alcalin, tel que le sodium ou le potassium, à titre d'agents tensioactifs.

Selon un mode de réalisation particulier, le système émulsionnant comprend au moins un agent tensioactif phosphaté, notamment le cétyl phosphate de potassium.

Selon un mode de réalisation particulier, le système émulsionnant comprend au moins un agent émulsionnant choisi parmi le stéareth-2, le stéarate de glycéryle, le stéarate de glycéryle polyéthoxylé à 30 groupes d'oxyde d'éthylène, le cétyl phosphate de potassium ou leurs mélanges.

Selon un mode de réalisation, le système émulsionnant de la composition selon l'invention comprend au moins un agent émulsionnant choisi parmi le cétyl phosphate de potassium, le stéareth-2, le stéareth-20 et leur mélange.

Selon un mode de réalisation, le système émulsionnant de la composition selon l'invention comprend du cétyl phosphate de potassium et du stéareth-2.

Selon un autre mode de réalisation, le système émulsionnant comprend un agent tensioactif de HLB supérieur à 8 en association avec un agent tensioactif de HLB inférieur à 8.

Selon un mode de réalisation, la composition selon l'invention comprend au moins un tensioactif anionique et au moins un tensioactif non ionique, en particulier un tensioactif non ionique de HLB infèrieur ou égal à 8 à 25 °C, lesdits tensioactifs pouvant être avantageusement choisis parmi les tensioactifs mentionnés ci-dessus.

La composition selon l'invention peut contenir de 0,01 à 30 % en poids d'agent émulsionnant, par rapport au poids total de ladite composition, mieux de 1 à 15 % en poids et mieux encore de 2 à 13 % en poids.

Selon un autre mode de réalisation, la composition selon l'invention comprend au moins un agent émulsionnant choisi parmi les esters d'acides gras et de polyol, notamment le stéarate de glycéryle, les esters d'acide gras et de polyéthylène glycol, notamment le stéarate de PEG-30, et leurs mélanges.

Co-tensioactifs

Selon un mode particulier de réalisation, les compositions selon l'invention peuvent comprendre en outre au moins un co-tensioactif autre que l'alcool béhénique.

Les co-tensioactifs peuvent notamment être choisis parmi les alcools gras, comprenant de préférence de 10 à 30 atomes de carbone. Par alcool gras comprenant de 10 à 30 atomes de carbone, on entend tout alcool gras pur, saturé ou non, ramifié ou non, comportant de 10 à 30 atomes de carbone.

A titre d'exemples d'alcools gras pouvant être utilisés en association avec le(s) agent(s) émulsiomiant(s) du système émulsionnant selon l'invention, on peut citer les alcools gras linéaires ou ramifiés, d'origine synthétique, ou encore naturelle comme par exemple les alcools provenant de matières végétales (coprah, palmiste, palme...) ou animales (suif...). Bien entendu, d'autres alcools à longue chaîne peuvent également être utilisés, comme par exemple les étheralcools ou bien encore les alcools dits de Guerbet. Enfin, on peut également utiliser certaines coupes plus ou moins longues d'alcools d'origine naturelle, comme par exemple coco ($C_{12}$ à $C_{16}$) ou suif ($C_{16}$ à $C_{18}$) ou des composés type diols ou cholesterol.

On utilise de préférence un alcool gras comprenant de 10 à 26 atomes de carbone, de préférence de 10 à 24 atomes de carbone, et plus préférentiellement de 12 à 21 atomes de carbone.

A titre d'exemples particuliers d'alcools gras utilisables dans le cadre de la présente invention, on peut notamment citer l'alcool laurique, myristique, cétylique, stéarylique, isostéarylique, palmitique, oléique, cétéarylique (mélange d'alcool cétylique et stéarylique), érucique, arachidylique et leurs mélanges.

De tels alcools gras sont notamment commercialisés sous la dénomination NAFOL par la société SASOL.

Parmi les co-tensioactifs utilisables selon l'invention, on peut également citer le mono et/ou distéarate de glycéryle.

Le ou les co-tensioactifs peuvent être présents en une teneur allant de 0,05 à 15 % en poids, de préférence de 0,1 à 10 % en poids, et plus préférentiellement de 1 à 8 % en poids par rapport au poids total de la composition.

Systèmes émulsionnants préférentiels

Selon un mode particulier de réalisation de l'invention, le système émulsionnant de la composition selon l'invention comprend au moins un agent tensioactif selon le point (i) énoncé précédemment et/ou au moins un agent tensioactif selon le point (iii) également énoncé ci-dessus, ainsi qu'éventuellement au moins un agent tensioactif selon le point (ii) et/ou au moins un agent tensioactif selon le point (iv) et/ou au moins un alcool gras comprenant de 10 à 26 atomes de carbone, de préférence de 10 à 24 atomes de carbone, et plus préférentiellement de 12 à 21 atomes de carbone.

Autrement dit, les modes de réalisation suivants sont particulièrement conformes à l'invention.

Selon un mode de réalisation particulier, ledit agent émulsionnant est un alkyle phosphate de métal alcalin ou oxyde de phosphine de formule $(R\text{-}O)_n$"P=O-$(O^-M)_m$ avec R représentant un groupe alkyle, linéaire ou ramifié, en $C_8$-$C_{22}$ tel que le cétyle, n étant égal à 1, 2 ou 3 et m étant égal à 0, 1 ou 2, avec m + n étant égal à 3 et M représentant un atome d'hydrogène ou un métal alcalin ou alcalino terreux, préférentiellement n = 1 et m = 2, et M est un métal alcalin, tel que le sodium ou le potassium.

Selon ce mode de réalisation, ledit agent émulsionnant est préférentiellement le cétyl phosphate de potassium.

Selon un autre mode de réalisation particulier, le système émulsionnant de la composition selon l'invention peut également comprendre, outre un alkyl phosphate de métal alcalin ou oxyde de phosphine décrit ci-dessus, un éther oxyéthyléné et/ou oxypropyléné (pouvant comporter de 1 à 150 groupes oxyéthyléné et/ou oxypropylénés) d'alcools gras (notamment d'alcool en $C_8$-$C_{24}$, et de préférence en $C_{12}$-$C_{18}$) tel que l'éther oxyéthyléné de l'alcool stéarylique à 2 groupes oxyéthylénés (nom CTFA « Stéareth-2 »).

Selon ce mode de réalisation, le système émulsionnant de la composition selon l'invention comprend préférentiellement le cétyl phosphate de potassium et du Stéareth-2.

Selon un mode de réalisation particulier, ledit agent émulsionnant est un sel d'acide glutamique de formule R-CONH-C($COO^-$M)-$C_2R_4$-COO-M' avec R représentant un groupe alkyle, linéaire ou ramifié en $C_8$-$C_{22}$ tel que le stéaryle et M' représentant un métal alcalin ou alcalino terreux.

Selon ce mode de réalisation, ledit agent émulsionnant est préférentiellement le glutamate de stéaroyle de sodium.

Selon un autre mode de réalisation particulier, le système émulsionnant de la composition selon l'invention peut également comprendre, outre un sel d'acide glutamique décrit ci-dessus, un alkyl glucoside obtenu par condensation

de glucose et d'alcools gras linéaires ou ramifiés en $C_8$-$C_{22}$ tel qu'un mélange cétylique et stéarique nommé cétylstéaryle.

Selon ce mode de réalisation, le système émulsionnant de la composition selon l'invention comprend préférentiellement du glutamate de stéaroyle de sodium et du glucoside de cétylstéaryle.

Selon un autre mode de réalisation particulier, le système émulsionnant de la composition selon l'invention peut comprendre un sel d'acide glutamique décrit ci-dessus et un alkyl phosphate de métal alcalin ou oxyde de phosphine également décrit ci-dessus.

Selon ce mode de réalisation, le système émulsionnant de la composition selon l'invention comprend préférentiellement du glutamate de stéaroyle de sodium et du cétyl phosphate de potassium.

Selon un autre mode de réalisation particulier, le système émulsionnant de la composition selon l'invention peut comprendre un sel d'acide glutamique, un alkyl phosphate de métal alcalin ou oxyde de phosphine et un éther oxyéthyléné et/ou oxypropyléné d'alcools gras comme décrits ci-dessus.

Selon ce mode de réalisation, le système émulsionnant de la composition selon l'invention comprend préférentiellement du glutamate de stéaroyle de sodium, du cétyl phosphate de potassium et du stéareth-2.

Selon un mode de réalisation particulier, le système émulsionnant de la composition selon l'invention comprend, outre un alkyl phosphate de métal alcalin ou oxyde de phosphine décrit ci-dessus, un co-tensioactif choisi parmi les alcools gras comprenant de 10 à 26 atomes de carbone, de préférence de 10 à 24 atomes de carbone, et plus préférentiellement de 12 à 21 atomes de carbone.

Selon ce mode de réalisation, le système émulsionnant de la composition selon l'invention comprend préférentiellement du cétyl phosphate de potassium et de l'alcool cétylique.

Selon un mode de réalisation particulier, le système émulsionnant de la composition comprend en outre au moins un éther oxyéthyléné et/ou oxypropyléné d'alcools gras comme décrit ci-dessus.

Selon ce mode de réalisation, le système émulsionnant de la composition selon l'invention comprend préférentiellement du cétyl phosphate de potassium, du stéareth-2 et de l'alcool cétylique.

Selon ce même mode de réalisation, le système émulsionnant de la composition selon l'invention comprend préférentiellement du cétyl phosphate de potassium, du stéareth-2, du stéareth-20 et de l'alcool cétylique.

Selon un mode de réalisation particulier, le système émulsionnant de la composition selon l'invention comprend un sel d'acide glutamique de formule R-CONH-C(COO-M)-$C_2H_4$-COO-M' avec R représentant un groupe alkyle, linéaire ou ramifié en $C_8$-$C_{22}$ tel que le stéaryle et M' représentant un métal alcalin ou alcalino terreux et un co-tensioactif choisi parmi les alcools gras comprenant de 10 à 26 atomes de carbone, de préférence de 10 à 24 atomes de carbone, et plus préférentiellement de 12 à 21 atomes de carbone.

Selon ce mode de réalisation préféré, le système émulsionnant de la composition selon l'invention comprend préférentiellement du glutamate de stéaroyle de sodium et de l'alcool cétylique.

Selon un mode de réalisation particulier, le système émulsionnant de la composition comprend en outre un alkyl glucoside obtenu par condensation de glucose et d'alcools gras linéaires ou ramifiés en $C_8$-$C_{22}$ tel qu'un mélange cétylique et stéarique nommé cétylstéaryle.

Selon ce mode de réalisation, le système émulsionnant de la composition selon l'invention comprend préférentiellement du glutamate de stéaroyle de sodium, du glucoside de cétylstéaryle et de l'alcool cétylique.

Enfin, selon un mode de réalisation particulier, le système émulsionnant de la composition comprend en outre un alkyl phosphate de métal alcalin ou oxyde de phosphine de formule $(R\text{-}O)_n\text{-}P{=}O\text{-}(OM)_m$ avec R représentant un groupe alkyle, linéaire ou ramifié, en $C_8$-$C_{22}$ tel que le cétyle, n étant égal à 1, 2 ou 3 et m étant égal à 0, 1 ou 2, avec m + n étant égal à 3 et M représentant un atome d'hydrogène ou un métal alcalin ou alcalino terreux, préférentiellement n = 1 et m = 2, et M est un métal alcalin, tel que le sodium ou le potassium.

Selon ce mode de réalisation, le système émulsionnant de la composition selon l'invention comprend préférentiellement du glutamate de stéaroyle de sodium, du cétyl phosphate de potassium et de l'alcool cétylique.

## MILIEU PHYSIOLOGIQUEMENT ACCEPTABLE

Les compositions selon l'invention peuvent comprendre un milieu physiologiquement acceptable, c'est-à-dire un milieu non toxique et susceptible d'être appliqué sur les fibres kératiniques d'êtres humains et d'aspect, d'odeur et de toucher agréables.

Le milieu physiologiquement acceptable est généralement adapté à la nature du support sur lequel doit être appliquée la composition, ainsi qu'à l'aspect sous lequel la composition doit être conditionnée.

Les compositions selon l'invention peuvent se présenter sous forme d'émulsion obtenue par dispersion d'une phase grasse dans une phase aqueuse et réalisée par voie directe ou indirecte.

Il peut s'agir d'émulsions simples obtenues par dispersion d'une phase grasse dans une phase aqueuse (H/E), ou d'émulsion de type émulsion multiple.

Selon un mode de réalisation particulier, la composition de l'invention se présente sous la forme d'une émulsion cire-dans-eau ou huile-dans-eau.

Les compositions de l'invention peuvent être de consistance liquide ou semi-liquide du type lait, ou de consistance molle, semi-solide ou solide de type crème ou gel.
Ces compositions sont préparées selon les méthodes usuelles.

## Phase aqueuse

La composition selon l'invention peut comprendre une phase aqueuse, qui forme la phase continue.
Par composition à phase continue aqueuse, on entend que la composition présente une conductivité, mesurée à 25 °C, supérieure ou égale à 23 μS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.
La phase aqueuse comprend de l'eau et/ou au moins un solvant hydrosoluble.
Par « solvant hydrosoluble », on désigne dans la présente invention un composé liquide à température ambiante et miscible à l'eau (miscibilité dans l'eau supérieure à 50 % en poids à 25 °C et pression atmosphérique).
Les solvants hydrosolubles utilisables dans les compositions selon l'invention peuvent en outre être volatils.
Parmi les solvants hydrosolubles pouvant être utilisés dans les compositions conformes à l'invention, on peut citer notamment les monoalcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3-butylène glycol et le dipropylène glycol, les cétones en $C_3$-$C_4$ et les aldéhydes en $C_2$-$C_4$.
La phase aqueuse (eau et éventuellement le solvant miscible à l'eau) est généralement présente dans la composition selon la présente demande en une teneur allant de 1 % à 80 % en poids, par rapport au poids total de la composition, de préférence allant de 10 % à 70 % en poids, préférentiellement allant de 15 % à 60 % en poids, et mieux de 30 % à 60% en poids.

## Phase grasse

La composition selon l'invention peut comprendre au moins une phase grasse liquide et/ou solide. Cette phase grasse peut comprendre au moins une cire, un corps gras pâteux, une huile ou leur mélange.
La phase grasse peut être présente dans une composition selon l'invention en une teneur allant de 1 % à 70 % en poids, par rapport au poids total de la composition, de préférence allant de 2 % à 50 % en poids, préférentiellement allant de 5 % à 40 % en poids, mieux allant de 15% à 40% en poids.
Selon un mode de réalisation préféré, une composition conforme à l'invention comprend en outre au moins un agent structurant lipophile tel que les cires, les corps gras pâteux et leurs mélanges.

### *Cires*

Les compositions selon l'invention peuvent comprendre éventuellement au moins une cire ou un mélange de cires. Ces cires peuvent être solides à température ambiante et à pression atmosphérique.
Les compositions selon l'invention, telles que les mascaras, peuvent comprendre une ou plusieurs cires, en une teneur allant de 1 % à 60 % en poids par rapport au poids total de la composition, notamment de 2 % à 45 % en poids, mieux allant de 15 % à 40 % en poids.
Selon un mode de réalisation particulier, une composition de l'invention peut notamment se présenter sous forme d'une émulsion cire-dans-eau, autrement dit comprendre une dispersion d'une cire ou mélange de cires dans une phase aqueuse continue.
La cire considérée dans le cadre de la présente invention est d'une manière générale un composé lipophile, solide à température ambiante (25 °C), à changement d'état solide/liquide réversible, ayant un point de fusion supérieur ou égal à 30°C pouvant aller jusqu'à 120 °C, à l'exception des alcools gras, tels que décrits précédemment, notamment des alcools gras présentant de 10 à 30 atomes de carbone et notamment de 12 à 22 atomes de carbone.
En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.
En particulier, les cires convenant à l'invention peuvent présenter un point de fusion supérieur à 45 °C environ, et en particulier supérieur à 55 °C.
Le point de fusion de la cire peut être mesuré à l'aide d'un calorimètre à balayage différentiel (D.S.C.), par exemple le calorimètre vendu sous la dénomination DSC 30 par la société METLER.
Le protocole de mesure est le suivant :

Un échantillon de 15 mg de produit disposé dans un creuset est soumis à une première montée en température allant de 0 °C à 120 °C, à la vitesse de chauffe de 10 °C/minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, déformables ou non à température ambiante, d'origine animale, végétale, minérale ou de synthèse et leurs mélanges.

La cire peut également présenter une dureté allant de 0,05 MPa à 30 MPa, et de préférence allant de 6 MPa à 15 MPa. La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :
La cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression maximale mesurée divisée par la surface du cylindre du texturomètre en contact avec la cire.

On peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en $C_8$-$C_{32}$.

Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut également utiliser les cires obtenues par transesterification et hydrogénation d'huiles végétales, telles que l'huile de ricin ou d'olive, comme les cires vendues sous les dénominations de Phytowax ricin 16L64® et 22L73® et Phytowax Olive 18L57 par la société SOPHIM. De telles cires sont décrites dans la demande FR-A-2792190.

On peut aussi utiliser des cires siliconées qui peuvent être avantageusement des polysiloxanes substitués, de préférence à bas point de fusion. Il s'agit notamment de polysiloxanes linéaires substitués constitués essentiellement (les groupes terminaux mis à part) de motifs de formules II et III, dans les proportions molaires respectives m et n :

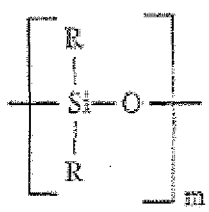

et

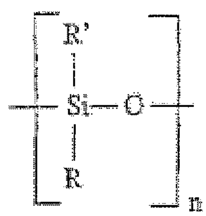

(II) (III)

dans lesquelles:

- chaque substituant R est défini comme précédemment,
- chaque R' représente indépendamment un alkyle (linéaire ou ramifié) éventuellement insaturé, ayant 6-30 atomes de carbone, ou bien un groupement -X-R", chaque X représentant indépendamment:

-O-,

$-(CH_2)_a-O-CO-$,

$-(CH_2)_b-CO-O-$,

a et b représentent indépendamment des nombres pouvant varier de 0 à 6, et
chaque R" représente indépendamment un groupement alkyles, éventuellement insaturé, ayant 6 à 30 atomes de carbone,

- m est un nombre pouvant varier de 0 à 400, et en particulier de 0 à 100,
- n est un nombre pouvant varier de 1 à 200, et en particulier de 1 à 100,

la somme (m + n) étant inférieure à 400, et en particulier inférieure ou égale à 100.

Ces cires de silicones sont connues ou peuvent être préparées selon les méthodes connues. Parmi les cires de silicones commerciales de ce type, on peut citer notament celles vendues sous les dénominations Abilwax 9800, 9801 ou 9810 (GOLDSCHMIDT), KF910 et KF7002 (SHIN ETSU), ou 176-1118-3 et 176-11481 (GENERAL ELECTRIC).
Les cires de silicone utilisables peuvent également être choisies parmi les composés de formule (IV) suivante :

$$R_1-Si(CH_3)_2-O-[Si(R)_2-O-]z-Si(CH_3)_2-R_2 \qquad (IV)$$

dans laquelle :

R est défini comme précédemment,
$R_1$ représente un groupement alkyle ayant de 1 à 30 atomes de carbone, un groupement alcoxy ayant de 6 à 30 atomes de carbone, ou un groupement de formule :

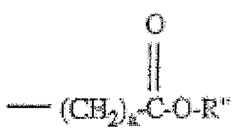 ou 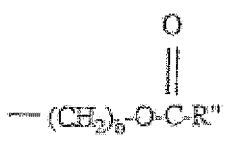

$R_2$ représente un groupement alkyle de 6 à 30 atomes de carbone, un groupement alcoxy ayant de 6 à 30 atomes de carbone ou un groupement de formule :

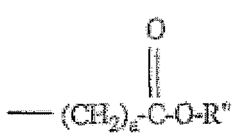 ou 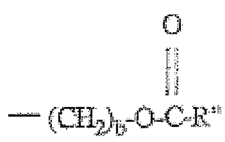

a et b représentant un nombre de 0 à 6,
R" étant un alkyle ayant de 6 à 30 atomes de carbone,
et z est un nombre pouvant varier de 1 à 100.

Parmi les cires de silicone de formole (IV), on citera notamment les alkyle ou alcoxydiméthicones tels que les produits commerciaux suivants : Abilwax 2428. 2434 et 2440 (GOLDSCHMIDT), ou VP 1622 et VP 1621 (WACKER), ainsi que les ($C_{20}$-$C_{60}$) alkyldiméthicones, en particulier les ($C_{30}$-$C_{45}$) alkyldiméthicones comme la cire siliconée vendue sous la dénomination SF-1642 par la société GE-Bayer Silicones.
On peut également utiliser des cires hydrocarbonées modifiées par des groupements siliconés ou fluorés comme par exemple : siliconyl candelilla, siliconyl beeswax et Fluorobeeswax de Koster Keunen.
Les cires peuvent également être choisies parmi les cires fluorées.
Selon un mode de réalisation particulier, les compositions selon l'invention peuvent comprendre au moins une cire dite cire collante c'est-à-dire possédant un collant supérieur ou égal à 0,7 N.s et une dureté inférieure ou égale à 3,5 MPa.
L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.
La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s

à 10 N.s, et en particulier allant de 2 N.s à 5 N.s.

Le collant de la cire est déterminé par la mesure de l'évolution de la force (force de compression ou force d'étirement) en fonction du temps, à 20 °C à l'aide du texturomètre vendu sous la dénomination « TA-TX2i® » par la société RHEO, équipé d'un mobile en polymère acrylique en forme de cône formant un angle de 45°.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.

Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.

La dureté est mesurée selon le protocole décrit précédemment.

Comme cire collante, on peut utiliser un (hydroxystçaryloxy)stéarate d'alkyle en $C_{20}$-$C_{40}$ (le groupe alkyle comprenant de 20 à 40 atomes de carbone), seul ou en mélange, en particulier un 12-(12'-hydroxystéaryloxy)stëarate d'alkyle en $C_{20}$-$C_{40}$.

Une telle cire est notamment vendue sous les dénominations « Kester Wax K 82 P® » et « Kester Wax K 80 P® » par la société KOSTER KEUNEN.

Les cires citées ci-dessus présentent généralement un point de fusion commençante inférieur à 45 °C.

La ou les cires peu(ven)t être présente(s) sous forme d'une microdispersion aqueuse de cire. On entend par microdispersion aqueuse de cire, une dispersion aqueuse de particules de cire, dans laquelle la taille, exprimée en diamètre « effectif » moyen en volume D[4,3], desdites particules de cire est inférieure ou égale à environ 1 μm.

Les microdispersions de cire sont des dispersions stables de particules colloïdales de cire, et sont notamment décrites dans «Microemulsions Theory and Practice », L.M. Prince Ed., Academic Press (1977) pages 21-32.

En particulier, ces microdispersions de cire peuvent être obtenues par fusion de la cire en présence d'un tensioactifs, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.

Les microdispersions de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 μm (notamment allant de 0,02 μm à 0,99 μm), de préférence inférieures à 0,5 μm (notamment allant de 0,06 μm à 0,5 μm).

Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

*Composées pâteux*

Les compositions selon l'invention, en particulier les compositions de type mascara peuvent comprendre en outre au moins un composé pâteux.

Par « composé pâteux » au sens de la présente invention, on entend un composé gras lipophile à changement d'état solide/liquide réversible et comportant à la température de 23 °C, une fraction liquide et une fraction solide.

En d'autres termes, la température de fusion commençante du composé pâteux est inférieure à 23 °C. La fraction liquide du composé pâteux, mesurée à 23 °C, représente de 20 à 97 % en poids du composé pâteux. Cette fraction liquide à 23 °C représente plus préférentiellement de 25 à 85 %, et mieux de 30 à 60 % en poids du composé pâteux.

La fraction liquide en poids du composé pâteux à 23 °C est égale au rapport de l'enthalpie de fusion consommée à 23°C sur l'enthalpie de fusion du composé pâteux.

L'enthalpie de fusion consommée à 23 °C est la quantité d'énergie absorbée par l'échantillon pour passer de l'état solide à l'état qu'il présente à 23 °C constitué d'une fraction liquide et d'une fraction solide.

L'enthalpie de fusion du composé pâteux est l'enthalpie consommée par le composé pour passer de l'état

solide à l'état liquide. Le composé pâteux est dit à l'état solide lorsque l'intégralité de sa masse est sous forme solide. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10 °C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

La fraction liquide du composé pâteux, mesurée à 32 °C, représente de préférence de 40 à 100 % en poids du composé pâteux, mieux encore de 50 à 100 % en poids du composé pâteux. Lorsque la fraction liquide du composé pâteux mesurée à 32 °C est égale à 100 %, la température de la fin de la plage de fusion du composé pâteux est inférieure ou égale à 32 °C.

La fraction liquide du composé pâteux, mesurée à 32 °C, est égale au rapport de l'enthalpie de fusion consommée à 32 °C sur l'enthalpie de fusion du composé pâteux. L'enthalpie de fusion consommée à 32 °C est calculée de la même façon que enthalpie de fusion consommée à 23 °C.

Le composé pâteux a de préférence une dureté à 20 °C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20 °C au centre de 5 échantillons. Le cylindre est introduit dans chaque échantillon, la profondeur de pénétration étant de 0,3 mm. La valeur relevée de la dureté est celle du pic maximum.

Le composé pâteux peut être choisi parmi les composés synthétiques et les composés d'origine végétale. Un composé pâteux peut être obtenu par synthèse à partir de produits de départ d'origine végétale.

Le composé pâteux est avantageusement choisi parmi :

- la lanoline et ses dérivés tels que l'alcool de lanoline, les lanolines oxyéthylénées, la lanoline acétylée, les esters de lanoline tels que le lanolate d'isopropyle, les lanolines oxypropylénées,
- les composés siliconés polymères ou non-polymères comme les polydiméthysiloxanes de masses moléculaires élevées, les polydiméthysiloxanes à chaînes latérales du type alkyle ou alcoxy ayant de 8 à 24 atomes de carbone, notamment les stéaryl diméthicones,
- les composés fluorés polymères ou non-polymères,
- les polymères vinyliques, notamment
- les homopolymères d'oléfines,
- les copolymère d'oléfines,
- les homopolymères et copolymères de diènes hydrogénés,
- les oligomères linéaires ou ramifiés, homo ou copolymères de (méth)acrylates d'alkyle ayant de préférence un groupement alkyle en $C_8$-$C_{30}$,
- les oligomères homo et copolymères d'esters vinyliques ayant des groupements alkyles en $C_8$-$C_{30}$,
- les oligomères homo et copolymères de vinyléthers ayant des groupements alkyles en $C_8$-$C_{30}$,
- les polyéthers liposolubles résultant de la polyéthérification entre un ou plusieurs diols en $C_2$-$C_{100}$, de préférence en $C_2$-$C_{50}$,
- les esters et les polyesters,
- et leurs mélanges.

Le composé pâteux peut être un polymère, notamment hydrocarboné.

Un composé pâteux siliconé et fluoré préféré est le polyméthyl-trifluoropropyl-méthylalkyl-diméthylsiloxane, fabriqué sous la dénomination X22-1088 par SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

Parmi les polyéthers liposolubles, on peut notamment citer les copolymères d'oxyde d'éthylène et/ou d'oxyde de propylène avec des oxydes d'alkylène en $C_6$-$C_{30}$. De préférence, le rapport pondéral de l'oxyde d'éthylène et/ou de l'oxyde de propylène avec les oxydes d'alkylène dans le copolymère est de 5:95 à 70:30. Dans cette famille, on citera notamment les copolymères blocs comprenant des blocs d'oxydes d'alkylène en $C_6$-$C_{30}$ ayant un poids moléculaire allant de 1 000 à 10 000, par exemple un copolymère bloc polyoxyéthylène/polydodécylène glycol tel que les éthers de dodécanediol (22 mol) et de polyéthylène glycol (45 motifs oxyéthylène ou OE) commercialisés sous la marque ELFACOS ST9 par Akzo Nobel.

Parmi les esters, on préfère notamment :

- les esters d'un glycérol oligomère, notamment les esters de diglycérol, en particulier les condensats d'acide adipique

et de glycérol, pour lesquels une partie des groupes hydroxyles des glycérols ont réagi avec un mélange d'acides gras comme l'acide stéarique, l'acide caprique, l'acide stéarique, l'acide isostéarique et l'acide 12-hydroxystéarique, tels que ceux notamment commercialisés sous la marque Softisan 649 par la société Sasol ;

- les esters de phytostérol ;
- les esters de pentaérythritol ;
- les esters formés à partir :
- d'au moins un alcool en $C_{16-40}$, l'un au moins des alcools étant un alcool de Guerbet et
- d'un dimère diacide formé à partir d'au moins un acide gras insaturé en $C_{18-40}$,

comme l'ester de dimère d'acides gras de tallol comprenant 36 atomes de carbone et d'un mélange i) d'alcools de Guerbet comprenant 32 atomes de carbone et ii) d'alcool béhénylique ; l'ester de dimère d'acide linoléique et d'un mélange de deux alcools de Guerbet, le 2-tétradécyl-octadécanol (32 atomes de carbone) et le 2-hexadccyl-eicosanol (36 atomes de carbone) ;

- les polyesters non réticulés résultant de la polycondensation entre un acide dicarboxylique ou un acide polycarboxylique, linéaire ou ramifié, en $C_4$-$C_{50}$, et un diol ou un polyol en $C_2$-$C_{50}$ ;
- les polyesters qui résultent de l'estérification entre un acide polycarboxylique et un ester d'acide carboxylique hydroxylé aliphatique comme le Risocast DA-L et le Risocast DA-H commercialisés par la société japonaise KOKYU ALCOHOL KOGYO, qui sont des esters résultant de la réaction d'estérification de l'huile de ricin hydrogénée avec l'acide dilinoléïque ou l'acide isostéarique ; et
- les esters aliphatiques d'ester résultant de l'estérification entre un ester d'acide carboxylique hydroxylé aliphatique et un acide carboxylique aliphatique, par exemple celui vendu sous la dénomination commerciale Salacos HCIS (V)-L par la société Nishing Oil.

Un alcool de Guerbet est le produit réactionnel de la réaction de Guerbet bien connue de l'homme du métier. Il s'agit d'une réaction transformant un alcool aliphatique primaire en son alcool dimère β-alkyle avec perte d'un équivalent d'eau.

Les acides carboxyliques aliphatiques décrits ci-dessus comprennent généralement de 4 à 30 et de préférence de 8 à 30 atomes de carbone. Ils sont choisis de préférence parmi l'acide héxanoïque, l'acide heptanoïque, l'acide octanoïque, l'acide 2-éthylhexanoïque, l'acide nonanoïque, l'acide décanoïque, l'acide undécanoïque, l'acide dodécanoïque, l'acide tridécanoïque, l'acide tétradécanoïque, l'acide pentadécanoïque, l'acide hexadécanoïque, l'acide hexyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

Les acides carboxyliques aliphatiques sont de préférence ramifiés.

Les esters d'acide carboxylique aliphatique hydroxylé sont avantageusement issus d'un acide carboxylique aliphatiques hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. Les esters d'acide carboxylique aliphatique hydroxylé sont notamment choisis parmi :

a) les esters, partiels ou totaux, d'acides monocarboxyliques aliphatiques monohydroxylés linéaires, saturés ;
b) les esters, partiels ou totaux, d'acides monocarboxyliques aliphatiques monohydroxylés insaturée ;
c) les esters, partiels ou totaux, de polyacides carboxyliques aliphatiques monohydroxylés saturés ;
d) les esters, partiels ou totaux, de polyacides carboxyliques aliphatiques polyhydroxylés saturés;
e) les esters, partiels ou totaux, de polyols aliphatiques en $C_2$ à $C_{16}$ ayant réagi avec un acide mono- ou un polycarboxylique aliphatique mono- ou polyhydroxylé,
f) et leurs mélanges.

Les esters aliphatiques d'ester sont avantageusement choisis parmi :

- l'ester résultant de la réaction d'estérification de l'huile de ricin hydrogénée avec l'acide isostéarique dans les proportions 1 pour 1 (1/1), qui est appelé monoisostéarate d'huile de ricin hydrogénée,
- l'ester résultant de la réaction d'estérification de l'huile de ricin hydrogénée avec l'acide isostéarique dans les proportions 1 pour 2 (1/2), qui est appelé le diisostéarate d'huile de ricin hydrogénée,
- l'ester résultant de la réaction d'estérification de l'huile de ricin hydrogénée avec l'acide isostéarique dans les proportions 1 pour 3 (1/3), qui est appelé le triisostéarate d'huile de ricin hydrogénée,
- et leurs mélanges.

De préférence, le composé pâteux est choisi parmi les composés d'origine végétale.

Parmi ceux-ci, on peut notamment citer l'huile de jojoba isomérisée telle que l'huile de jojoba partiellement

hydrogénée isomérisée tyrans fabriquée ou commercialisée par la société Desert Whale sous la référence commerciale Iso-Jojoba-50®, la cire d'orange comme, par exemple, celle qui est commercalisée sous la référence Orange Peel Wax par la société Koster Keuner2, le beurre de cupuacu (Rain forest RF3410), le beurre de murumuru (murumuru butter de la société Beraca Sabara), le beurre de karité, l'huile d'olive partiellement hydrogénée comme, par exemple, le composé commercialisé sous la référence Beurrolive par la société Soliance, le beurre de cacao, l'huile de mangue comme, par exemple, la Lipex 302 de la société Aarhuskarlshamn.

Selon un mode de réalisation particulier, une composition selon l'invention comprend du beurre de karité.

Le ou les composés pâteux peuvent être présents en une quantité supérieure allant de 0,1 % à 20 % en poids, notamment de 0,5 % à 10 % en poids, par rapport au poids total de la composition.

La phase grasse d'une composition selon l'invention peut le cas échéant éventuellement comprendre au moins une ou plusieurs huiles ou solvants organiques.

*Huiles ou solvants organiques*

Par « huile ou solvant organique », au sens de la demande, on entend un corps non aqueux liquide à température ambiante (25 °C) et pression atmosphérique (760 mm de Hg).

L'huile peut être choisie parmi les huiles volatiles et/ou les huiles non volatiles, et leurs mélanges.

La ou les huiles peuvent être présentes en une teneur allant de 1 % à 50 % en poids, de préférence de 5 % à 30 % en poids par rapport au poids total de la composition.

Par « huile volatile », on entend au sens de l'invention une huile susceptible de s'évaporer au contact des fibres kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg).

Par « huile non volatile », on entend une huile restant sur les fibres kératiniques à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

<u>Huile volatile</u>

La composition selon l'invention peut comprendre au moins une huile volatile. Cette huile volatile peut être hydrocarbonée. L'huile volatile hydrocarbonée peut être choisie parmi les huiles hydrocarbonées ayant de 7 à 16 atomes de carbone. L'huile volatile hydrocarbonée peut être présente dans la composition selon l'invention en une teneur allant de 0,1 % à 90 % en poids, par rapport au poids total de la composition, de préférence allant de 1 % à 70 % en poids, et préférentiellement allant de 5 % à 70 % en poids, voire de 5 % à 50 % en poids.

La composition selon l'invention peut contenir un ou plusieurs alcane(s) ramifié(s) volatil(s). Par « un ou plusieurs alcane(s) ramifié(s) volatil(s) », on entend indifféremment « une ou plusieurs huile alcane(s) ramifié(s) volatile(s) ».

Comme huile volatile hydrocarbonée ayant de 7 à 16 atomes de carbone, on peut citer notamment les alcanes ramifiés en $C_8$-$C_{16}$ comme les iso-alcanes (appelées aussi isoparaffines) en $C_8$-$C_{16}$, l'isododécane, l'isodécane, l'isohexadécane et par exemple les huiles vendues sous les noms commerciaux d'Isopars ou de Permetyls, les esters ramifiés en $C_8$-$C_{16}$ comme le néopentanoate d'iso-hexyle, et leurs mélanges. De préférence, l'huile volatile hydrocarbonée ayant de 8 à 16 atomes de carbone est choisie parmi l'isododécane, l'isodécane, l'isohexadécane et leurs mélanges, et est notamment l'isododécane.

La composition selon l'invention peut contenir un ou plusieurs alcane(s) linéaire(s) volatil(s). Par « un ou plusieurs alcane(s) linéaire(s) volatil(s) », on entend indifféremment « une ou plusieurs huile alcane(s) linéaire(s) volatile (s) ».

Un alcane linéaire volatil convenant à l'invention est liquide à température ambiante (environ 25 °C) et à la pression atmosphérique (760 mm Hg).

Par « alcane linéaire volatil » convenant à l'invention, on entend un alcane linéaire cosmétique, susceptible de s'évaporer au contact de la peau en moins d'une heure, à température ambiante (25 °C) et pression atmosphérique (760 mm Hg, c'est-à-dire 101 325 Pa), liquide à température ambiante, ayant notamment une vitesse d'évaporation allant de 0,01 à 15 $mg/cm^2/min$, à température ambiante (25 °C) et pression atmosphérique (760 mm Hg).

De façon préférée, les « alcanes linéaires volatils » convenant à l'invention présentent une vitesse d'évaporation allant de 0,01 à 3,5 $mg/cm^2/min$, à température ambiante (25 °C) et pression atmosphérique (760 mm Hg).

De façon préférée, les « alcanes linéaires volatils » convenant à l'invention présentent une vitesse d'évaporation

allant de 0,01 à 1,5 mg/cm2/min, à température ambiante (25 °C) et pression atmosphérique (760 mm Hg).

De façon plus préférée, les « alcanes linéaires volatils » convenant à l'invention présentent une vitesse d'évaporation allant de 0,01 à 0,8 mg/cm2/min, à température ambiante (25 °C) et pression atmosphérique (760 mn Hg).

De façon encore préférée, les « alcanes linéaires volatils » convenant à l'invention présentent une vitesse d'évaporation allant de 0,01 à 0,3 mg/cm2/min, à température ambiante (25 °C) et pression atmosphérique (760 mm Hg).

De façon encore préférée, les « alcanes linéaires volatils convenant à l'invention présentent une vitesse d'évaporation allant de 0,01 à 0,12 mg/cm2/min, à température ambiante (25 °C) et pression atmosphérique (760 mm Hg).

La vitesse d'évaporation d'un alcane volatil conforme à l'invention (et plus généralement d'un solvant volatil) peut être notamment évaluée au moyen du protocole décrit dans WO 061013413, et plus particulièrement au moyen du protocole décrit ci-après.

On introduit dans un cristallisoir (diamètre : 7 cm) placé sur une balance se trouvant dans une enceinte d'environ 0,3 m3 régulée en température (25 °C) et en hygrométrie (humidité relative 50 %) 15 g de solvant hydrocarboné volatil.

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm2) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm2) et par unité de temps (minute).

Selon un mode de réalisation préféré, les « alcanes linéaires volatils » convenant à l'invention ont une pression de vapeur (appelée également pression de vapeur saturante) non nulle, à température ambiante, en particulier une pression de vapeur allant de 0,3 Pa à 6000 Pa.

De façon préférée, les « alcanes linéaires volatils » convenant à l'invention ont une pression de vapeur allant de 0,3 à 2000 Pa, à température ambiante (25 °C).

De façon préférée, les « alcanes linéaires volatils » convenant à l'invention ont une pression de vapeur allant de 0,3 à 1000 Pa, à température ambiante (25 °C).

De façon plus préférée, les « alcanes linéaires volatils » convenant à l'invention ont une pression de vapeur allant de 0,4 à 600 Pa, à température ambiante (25 °C).

De façon préférée, les « alcanes linéaires volatils » convenant à l'invention ont une pression de vapeur allant de 1 à 200 Pa, à température ambiante (25 °C).

De façon encore préférée, les « alcanes linéaires volatils » convenant à l'invention ont une pression de vapeur allant de 3 à 60 Pa, à température ambiante (25 °C).

Selon un mode de réalisation, un alcane linéaire volatil convenant à l'invention peut présenter un point éclair compris dans l'intervalle variant de 30 à 120 °C, et plus particulièrement de 40 à 100 °C. Le point éclair est en particulier mesuré selon la Norme iso 3679.

Selon un mode de réalisation, un alcane convenant à l'invention peut être un alcane linéaire volatil comprenant de 7 à 14 atomes de carbone.

De façon préférée, les « alcanes linéaires volatils » convenant à l'invention comprennent de 8 à 14 atomes de carbone.

De façon préférée, les « alcanes linéaires volatils » convenant à l'invention comprennent de 9 à 14 atomes de carbone.

De façon préférée, les « alcanes linéaires volatils » convenant à l'invention comprennent de 10 à 14 atomes de carbone.

De façon préférée, les « alcanes linéaires volatils » convenant à l'invention comprennent de 11 à 14 atomes de carbone.

Selon un mode de réalisation avantageux, les « alcanes linéaires volatils » convenant à l'invention présentent une vitesse d'évaporation, telle que définie plus haut, allant de 0,01 à 3,5 mg/cm2/min, à température ambiante (25°C) et pression atmosphérique (760 mm Hg), et comprennent de 8 à 14 atomes de carbone.

Un alcane linéaire volatil convenant à l'invention peut être avantageusement d'origine végétale.

De préférence, l'alcane linéaire volatil ou le mélange d'alcanes linéaires volatils présent dans la composition selon l'invention comprend au moins un isotope $^{14}C$ du carbone (carbone 14), en particulier l'isotope $^{14}C$ peut être présent en un ratio $^{14}C$ / supérieur ou égal à $1.10^{-16}$, de préférence supérieur ou égal à $1.10^{-15}$, de préférence encore supérieur ou égal $7,5.10^{-14}$, et mieux supérieur ou égal $1,5.10^{-13}$. De préférence, le ratio $^{14}C/^{12}C$ va de $6.10^{-13}$ à $1,2.10^{-12}$.

La quantité de d'isotopes $^{14}C$ dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes convenant à l'invention, on peut mentionner les alcanes décrits dans les demandes de brevets de la société Cognis WO 20071068371, ou W02008/155059 (mélanges d'alcanes distincts et différant d'au moins un carbone). Ces alcanes sont obtenus partir d'alcools gras, eux-mêmes obtenus à partir d'huile de coprah ou de palme.

A titre d'exemple d'alcanes linéaires convenant à l'invention, on peut citer le n- heptane (C7), le n-octane (C8), le n-nonane (C9), le n-décane (C10), le n-undécane (C11), le n-dodécane (C12), le n-tridécane (C13), le n-tétradecane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane (C11) et de n-tridécane (C13) obtenus aux exemples 1 et 2 de la demande WO2008/15505 de la Société Cognis.

On peut également citer le n-dodécane (C12) et le n-tétradécane (C14) vendus par Sasol respectivement sous les références PARAFOL 12-97 et PARAFOL 14-97, ainsi que leurs mélanges.

On pourra utiliser l'alcane linéaire volatil seul.

On pourra alternativement ou préférentiellement utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un premier mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils $C_{10}/C_{11}$, $C_{11}/C_{12}$, ou $C_{12}/C_{13}$.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils $C_{10}/C_{12}$, ou $C_{12}/C_{14}$, pour un nombre de carbone n pair et le mélange $C_{11}/C_{13}$ pour un nombre de carbone n impair.

Selon un mode préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils $C_{11}/C_{13}$ ou un mélange d'alcanes linéaires volatils $C_{12}/C_{14}$.

D'autres mélanges associant plus de 2 alcanes linéaires volatils selon l'invention, tels que par exemple un mélange d'au moins 3 alcanes linéaires volatils comportant de 7 à 14 atomes de carbone distincts et différant entre eux d'un nombre de carbone d'au moins 1, font également partie de l'invention, mais les mélanges de 2 alcanes linéaires volatils selon l'invention sont préférés (mélanges binaires), lesdits 2 alcanes linéaires volatils représentant de préférence plus de 95 % et mieux plus de 99 % en poids de la teneur totale en alcanes linéaires volatils dans le mélange. Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :

- de 50 à 90 % en poids, de préférence de 55 à 80 % en poids, préférentiellement encore de 60 à 75 % en poids d'alcane linéaire volatil en Cn avec n allant de 7 à 14,
- de 10 à 50 % en poids, de préférence de 20 à 45 % en poids, de préférence de 24 à 40% en poids, d'alcane linéaire volatil en Cn+x avec x supérieur ou égal à 1, de préférence x=1 ou x=2, avec n+x compris entre 8 et 14, par rapport au poids total des alcanes dans ledit mélange.

En particulier, ledit mélange d'alcanes selon l'invention contient :

- moins de 2 % en poids, de préférence moins de 1 % en poids d'hydrocarbures ramifiés,
- et/ou moins de 2 % en poids, de préférence moins de 1 % en poids d'hydrocarbures aromati ques,
- et/ou moins de 2 % en poids, de préférence moins de 1% en poids et préférentiellement moins de 0,1 % en poids d'hydrocarbures insaturés dans le mélange.

Plus particulièrement, un alcane linéaire volatil convenant à l'invention peut être mis en oeuvre sous la forme d'un mélange n-undécanc/n-tridécane,

En particulier, on utilisera un mélange d'alcanes linéaires volatils comprenant :

- de 55 à 80 % en poids, de préférence de 60 à 75 % en poids d'alcane linéaire volatil en C11 (n-undécane)

- de 20 à 45 % en poids, de préférence de 24 à 40 % en poids d'alcane linéaire volatil en $C_{13}$ (n-tridécane) par rapport au poids total des alcanes dans ledit mélange.

Selon un mode de réalisation particulier, le mélange d'alcanes est un mélange n-undécaneln-tridécane. En particulier un tel mélange peut être obtenu selon l'exemple 1 ou l'exemple 2 du WO 2008/155059.
Selon un autre mode de réalisation particulier, on utilise le n-dodécane vendu sous la référence PARAFOL 12-97 par SASOL.
Selon un autre mode de réalisation particulier, on utilise le n-tétradëcane vendu sous la référence PARAFOL 14-97 par SASOL.
Selon encore un autre mode de réalisation, on utilise un mélange de n-dodécane et de n-tétradécane.
En variante ou de façon additionnelle, la composition réalisée peut comprendre au moins une huile ou solvant siliconée volatile, compatible avec une utilisation cosmétique.
Par huile siliconée, on entend une huile contenant au moins un atome de silicium, et notamment contenant des groupes Si-O. Selon un mode de réalisation, ladite composition comprend moins de 10 % en poids d'huile(s) siliconée (s) non volatiles, par rapport au poids total de la composition, mieux moins de 5 % en poids, voire est exempte d'huile siliconée.
Comme huile volatile siliconée, on peut citer les polysiloxanes cycliques, les polysiloxanes linéaires et leurs mélanges. Comme polysiloxanes volatiles linéaires, on peut citer l'hexamethyldisiloxane, l'octamethyltrisiloxane, le decamethyltetrasiloxane, le tetradecamethylhexasiloxane et l'hexadecamethylheptasiloxane. Comme polysiloxanes volatiles cycliques, on peut citer l'hexamethylcyclotrisiloxane, l'octamethylcylotetrasiloxane, le decamethylcyclopentasiloxane et le dodecamethylcyclohexasiloxane.
En variante ou de façon additionnelle, la composition réalisée peut comprendre au moins une huile volatile fluorée.
Par huile fluorée, on entend une huile contenant au moins un atome de fluor.
Comme huile volatile fluorée, on peut citer le nonafluorométhoxybutane ou le perfluorométhylcyclopentane, et leurs mélanges.

_Huiles non volatiles_

Les huiles non volatiles peuvent, notamment, être choisies parmi les huiles hydrocarbonées, fluorées et/ou les huiles siliconées non volatiles.
Comme huile hydrocarbonée non volatile, on peut notamment citer :

- les huiles hydrocarbonées d'origine animale,
- les huiles hydrocarbonées d'origine végétale, telles que les esters de phytostéaryle, tels que l'oléate de phytostéaryle, l'isostéarate de physostéaryle et le glutamate de lauroyl/octyldodécyle/phytostéaryle (AJINOMOTO, ELDEW PS203), les triglycérides constitués d'esters d'acides gras et de glycérol, en particulier, dont les acides gras peuvent avoir des longueurs de chaînes variant de $C_4$ à $C_{36}$, et, notamment, de $C_{18}$ à $C_{36}$; ces huiles pouvant être linéaires ou ramifiées, saturées ou insaturées ; ces huiles peuvent, notamment, être des triglycérides héptanoïques ou octanoïques, l'huile de karité, de luzerne, de pavot, de potimarron, de millet, d'orge, de quinoa, de seigle, de bancoulier, de passiflore, le beurre de karité, l'huile d'aloès, l'huile d'amande douce, l'huile d'amande de pêche, l'huile d'arachide, l'huile d'argan, l'huile d'avocat, l'huile de baobab, l'huile de bourrache, l'huile de brocoli, l'huile de calendula, l'huile de caméline, l'huile de carotte, l'huile de carthame, l'huile de chanvre, l'huile de colza, l'huile de coton, l'huile de coprah, l'huile de graine de courge, l'huile de germe de blé, l'huile de jojoba, l'huile de lys, l'huile de macadamia, l'huile de maïs, l'huile de meadowfoam, l'huile de millepertuis, l'huile de monoï, l'huile de noisette, l'huile de noyaux d'abricot, l'huile de noix, l'huile d'olive, l'huile d'onagre, l'huile de palme, l'huile de pépins de cassis, l'huile de pépins de kiwi, l'huile de pépins de raisin, l'huile de pistache, l'huile de potimarron, l'huile de potiron, l'huile de quinoa, l'huile de rosier muscat, l'huile de sésame, l'huile de soja, l'huile de tournesol, l'huile de ricin, et l'huile de watermelon, et leurs mélanges, ou encore des triglycérides d'acides caprylique/caprique, comme ceux vendus par la société STEARINERIES DUB0IS ou ceux vendus sous les dénominations MIGLYOL 810®, 812® et 818® par la société DYNAMIT NOBEL,
- les éthers de synthèse ayant de 10 à 40 atomes de carbone ;
- les esters de synthèse, comme les huiles de formule $R_1COOR_2$ dans laquelle $R_1$ représente un reste d'un acide gras linéaire ou ramifié comportant de 1 à 40 atomes de carbone et $R_2$ représente une chaîne hydrocarbonée, notamment, ramifiée contenant de 1 à 40 atomes de carbone à condition que $R_1 + R_2$ soit ≥ 10.Les esters peuvent être, notamment, choisis parmi les esters d'alcool et d'acide gras, comme par exemple :

  · l'octanoate de cétostéaryle, les esters de l'alcool isopropylique, tels que le myristate d'isopropyle, le palmitate

d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés, comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools, comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylène glycol et leurs mélanges, les benzoates d'alcools en $C_{12}$-$C_{I5}$, le laurate d'hexyle, les esters de l'acide néopentanoïque, comme le néopentanoate d'isodécyle, le néopentanoate d'isotridécyle, le néopentanoate d'isostéaryle, le nëopentanoate d'octyldocécyle, les esters de l'acide isononanoïque, comme l'isononanoate d'isononyle, l'isononanoate d'isotridécyle, l'isononanoate d'octyle, les esters hydroxylés comme le lactate d'isostéaryle, le malate de di-isostéaryle ;

- les esters de polyols et les esters du pentaérythritol, comme le tétrahydroxystéarate/tétraisostéarate de dipentaérythritol,
- les esters de dimères diols et de dimères diacides, tels que les Lusplan DD-DA5® et Lusplan DD-DA7®, commercialisés par la société NIPPON FINE CHEMICAL et décrits dans la demande US 2004-175338,
- les copolymères de dimère diol et de dimère diacide et leurs esters, tels que les copolymères dimères dilinoleyl diol/dimères dilinoléiques et leurs esters, comme par exemple le Plandool-G,
- les copolymères de polyols et de dimères diacides, et leurs esters, tels que le Hailuscent ISDA,
- les alcools gras liquides à température ambiante à chaîne carbonée ramifiée et/ou insaturée ayant de 12 à 26 atomes de carbone, comme le 2-octyldodécanol, l'alcool isostéarylique, l'alcool oléique, le 2-hexyldécanol, le 2-blatyloctanol, et le 2-undécylpentadécanol,
- les acides gras supérieurs en $C_{12}$-$C_{22}$, tels que l'acide oléique, l'acide linoléique, l'acide linoléique et leurs mélanges, et
- les carbonates de di-alkyle, les 2 chaînes alkyles pouvant être identiques ou différentes, tels que le dicaprylyl carbonate commercialisé sous la dénomination CETIOL CC®, par COGNIS,
- les huiles de masse molaire élevée ayant, en particulier, une masse molaire allant d'environ 400 à environ 10 000 g/mol, en particulier, d'environ 650 à environ 10 000 g/mol, en particulier, d'environ 750 à environ 7500 g/mol, et plus particulièrement, variant d'environ 1000 à environ 5000 g/mol. Comme huile de masse molaire élevée utilisable dans la présente invention, on peut notamment citer les huiles choisies parmi :

  . les polymères lipophiles,
  . les esters d'acides gras linéaires ayant un nombre total de carbone allant de 35 à 70.
  . les esters hydroxylés,
  . les esters aromatiques,
  . les esters d'alcools gras ou d'acides gras ramifiés en $C_{24}$-$C_{28}$,
  . les huiles siliconées,
  . les huiles d'origine végétale,
  . et leurs mélanges.

Par exemple, une huile de masse molaire élevée peut être choisie parmi :

a) les polymères lipophiles, tels que :

. les polybutylènes, tels que L'INDOPOL H-100 (de masse molaire MM=965 g/mol), L'INDOPOL H-300 (MM=1340 g/mol), L'INDOPOL H-1500 (MM=2160g/mol) commercialisés ou fabriqués par la société AMOCO,
. les polyisobutylènes, par exemple, hydrogénés, tels que le PANALANE H-300 E commercialisés ou fabriqué par la société AMOCO (MM =1340 glrnol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
. les polydécènes et les polydécènes hydrogénés, tels que : le PURESYN 10 (MM=723 g/mol), le PURESYN 150 (MM=9200 g/mol) commercialisés ou fabriqués par la société MOBIL CHEMICALS,
. les copolymères de la vinylpyrrolidone, tels que : le copolymère vinylpynolidone/1-hexadécène, ANTARON V-216 commercialisé ou fabriqué par la société ISP (MM=7300 g/mol), et les copolymères de palyvinylpyrrolidone (PVP), tels que les copolymères d'un alcène en $C_2$-$C_{30}$, tel qu'en $C_3$-$C_{22}$, et des associations de ceux-ci, peuvent être utilisés. Comme exemples de copolymères de PVP pouvant être utilisés dans l'invention, on peut citer le copolymère de PVP/laurate de vinyle, de PVP/stéarate de vinyle, la PVP butylée, de PVP/hexadécène, de PVP/triacontène ou de PVP/acide acrylique/méthacrylate de lauryle,

b) les esters, tels que :

. les esters d'acides gras linéaires ayant un nombre total de carbone allant de 35 à 70, comme le tétrapélargonate de pentaérythrityle (MM=697 g/mol),

. les esters hydroxylés, tels que le triisostéarate de polyglycérol-2 (MM=965 g/mol),

. les esters aromatiques, tels que le tridécyl trimellitate (MM=757 g/mol),

. les esters d'alcools gras ou d'acides gras ramifiés en $C_{24}$-$C_{28}$, tels que ceux décrits dans le brevet US 6,491,927 et les esters du pentaérythritol, et, notamment, le citrate de triisoarachidyle (MM=1033,76 g/mol), le tétraisononanoate de pentaérythrityle (MM=697 g/mol), le triisostéarate de glycéryle (MM=891 g/mol), le tri décyl-2 tétradécanoate de glycéryle (MM=1143 g/mol), le tétraisostéarate de pentaérythrityle (MM=1202 g/mol), le tétraisostéarate de polyglycéryle -2 (MM=1232 g/mol) ou encore le tétra décyl-2 tétradécanoate de pentaérythrityle (MM=1538 g/mol),

. les esters et polyesters de dimère diol, tels que les esters de dimère diol et d'acide gras, et les esters de dimère diols et de diacide, tels que les Lusplan DD-DA5® et Lusplan DD-DA7® commercialisés par la société NIPPON FINE CHEMICAL et décrits dans la demande US 2004-175338,

c) les huiles siliconées, telles que les silicones phénylées comme la BELSIL PDM 1000 de la société WACIER (MM=9000 g/mol). D'autres huiles de silicone non volatiles utilisables dans la composition selon l'invention peuvent être les polydiméthylsiloxanes (PDMS) non volatiles, les PDMS comportant des groupements alkyle ou alcoxy pendants et/ou en bouts de chaîne siliconée, groupements ayant chacun de 2 à 24 atomes de carbone, les silicones phénylées, comme les phényl triméthicones, les phényl diméthicones, les phénol triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicone de viscosité inférieure ou égale à 100 cSt, et leurs mélanges,
ainsi que les mélanges des huiles a) et/ou b) et/ou c).

Les huiles fluorées utilisables dans l'invention sont notamment des huiles fluorosiliconées, des polyéthers fluorés, des silicones fluorées telles que décrit dans le document EP-A-847752.

Les compositions selon l'invention peuvent en outre comprendre tout ingrédient classiquement utilisé dans les domaines concernés et plus spécialement dans le domaine des mascaras et/ou des vernis à ongles, tels que par exemple des pigments ou nacres, des polymères filmogènes, des gélifiants, des charges et/ou des fibres.

## Pigments

Par « pigments », il faut comprendre des particules blanches ou colorées, minérales ou organiques, insolubles dans un milieu aqueux, destinées à colorer et/ou opacifier la composition et/ou le film résultant.

Les pigments peuvent être blancs ou colorés, minéraux et/ou organiques.

Le pigment peut être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références C1 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI11725, 15510,45370, 71105,les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Ces pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

Le pigment peut aussi être une laque. Par laque, on entend les colorants insolubilisés adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants organiques, on peut citer le carmin de cochenille. On peut également citer les produits connus sous les dénominations suivantes : D & C Red 21 (CI 45 380), D & C Orange 5 (CI 45 370), D & C Red 27 (CI

45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985). D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, le dioxyde de titane, les poudres métalliques comme la poudre d'aluminium et la poudre de cuivre. Les pigments minéraux suivants peuvent aussi être utilisés : $Ta_2O_5$, $Ti_3O_5$, $Ti_2O_3$, TiO, $ZrO_2$ en mélange avec $TiCO_2$, $ZrO_2$, $Nb_2O_5$, $CeO_2$, ZnS.

La taille du pigment utile dans le cadre de la présente invention est généralement comprise entre 10 nm et 10 $\mu$m, de préférence entre 20 nm et 5 $\mu$m, et plus préférentiellement entre 30 nm et 1 $\mu$m.

**Polymères filmogènes**

Parmi les polymères filmogènes utilisables dans les compositions de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styréniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styréniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amine alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ ou un ion métallique, comme par exemple un ion $Na^+$, $Li^+$, $K^+$, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl, sulfonyldiphényl. méthylènediphényl. On peut citer comme exemple de monomère aromatique bifonctionnel portant en outre un groupement $-SO_3M$ : l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

Les polymères d'origine naturelle, éventuellement modifiées, peuvent être choisis parmi la résine shellac, la gomme de sandaraque, les dammars, les élémis, les copals, les polymères cellulosiques, et leurs mélanges.

Selon un premier mode de réalisation de l'invention, le polymère filmogène peut être un polymère hydrosoluble et peut être alors présent dans la phase continue aqueuse d'une émulsion selon l'invention.

Selon une autre variante, le polymère filmogène peut être un polymère solubilisé dans une phase grasse liquide comprenant des huiles ou solvants organiques tels que ceux décrits par la suite (on dit alors que le polymère filmogène est un polymère liposoluble). De préférence, la phase grasse liquide comprend une huile volatile, éventuellement en mélange avec une huile non volatile, les huiles pouvant être choisies parmi les huiles citées ci-dessous.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyle éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux allyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de méthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2 000 à 500 000 et de préférence de 4 000 à 200000.

On peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 2 à 24 atomes de carbone.

Comme exemples d'homopolymères liposolubles, on peut citer notamment: les polylaurate de vinyle et le poly (méth)acrylates de lauryle, ces poly(méth)acrylates pouvant être réticulés à l'aide de diméthacrylate de l'éthylène glycol ou de tétraéthylène glycol.

Selon un mode de réalisation avantageux, une composition selon l'invention comprend au moins un polymère filmogène polylaurate de vinyle.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la vinylpyrrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécene, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de « MDTQ », la résine étant décrite en fonction des différentes unités monomériques siloxane qu'elle comprend, chacune des lettres « MT3TQ » caractérisant un type d'unité.

A titre d'exemples de résines polyméthylsilsesquioxanes commercialement disponibles, on peut citer celles qui sont commercialisés par la société Wacker sous la référence Resin MK tels que la Belsil PMS MK, et par la société SHIN-ETSU sous les références KR-220L.

Comme résines siloxysilicates, on peut citer les résines triméthylsiloxysilicate (TMS) telles que celles commercialisées sous la référence SR1000 par la société General Electric ou sous la référence TMS 803 par la société Wacker. On peut encore citer les résines triméthylsiloxysilicate commercialisées dans un solvant tel que la cyclomethicone, vendues sous la dénomination « KF-7312J » par la société Shin-Etsu, «DC 749 », « DC 593 » par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Coing sous la référence BIO-PSA et décrits dans le document US 5,162,410 ou encore les copolymères siliconés issus de la réaction d'un résine de silicone, telle que celles décrite plus haut, et d'un diorganosiloxane tels que décrits dans le document WO 2004/073626.

On peut également utiliser les polyamides siliconés du type polyorganosiloxane tels que ceux décrits dans les documents US-A-5.,874,069, US-A-5,919,441, US-A-6,051,216 et US-A-5,981,680.

Ces polymères siliconés peuvent appartenir aux deux familles suivantes :

- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

Selon un mode de réalisation de l'invention, le polymère filmogènes est un polymère éthylénique séquencé linéaire filmogènes, qui comprend de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Avantageusement, les première et deuxième séquences et du polymère séquencé sont incompatibles l'une avec l'autre.

De tels polymères sont décrits par exemple dans les documents EP 1 411 069 ou WO 04/028488.

Le polymère filmogène peut être également présent dans une composition de l'invention sous la forme de

particules en dispersion dans une phase aqueuse ou dans une phase solvant non aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO ; Syntran 5760® par la société Interpolymer, Allianz OPT par la société ROHM & HAAS, les dispersions aqueuses de polymères acryliques ou styrène/acrylique vendues sous le nom de marque JONCRYL® par la société JOHNSON POLYMER ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425® Avalure UR-450®, Sancure 875®, Sancure 861®, Sancure 878® et Sancure 2060® par la société GOODRICH, Impranil 85® par la société BAYER, Aquamere H-1511® par la société HYDROMER ; les sulfopolyesters vendus sous le nom de marque Eastman AQ® par la société Eastman Chemical Products, les dispersions vinyliques comme le Mexomère PAM® de la société CHLMEX et leurs mélanges.

Comme exemples de dispersions non aqueuses de polymère filmogène, on peut citer les dispersions acryliques dans l'isododécane comme le Mexomère PAP® de la société CHIMEX, les dispersions de particules d'un polymère éthylénique greffé, de préférence acrylique, dans une phase grasse liquide, le polymère éthylénique étant avantageusement dispersé en l'absence de stabilisant additionnel en surface des particules telles que décrite notamment dans le document WO 04/055081.

Une composition selon l'invention peut également comprendre en outre un agent plastifiant favorisant la formation d'un film avec le polymère filmogène. Un tel agent plastifiant peut être choisi parmi tous les composés connus de l'homme du métier comme étant susceptibles de remplir la fonction recherchée.

**<u>Gélifiants</u>**

Une composition de l'invention peut également comprendre au moins un gélifiant hydrophile ou hydrosoluble.

Comme gélifiants hydrophiles ou hydrosolubles, on peut citer:

- les homo- ou copolymères d'acides acrylique ou méthacrylique ou leurs sels et leurs esters et en particulier les produits vendus sous les dénominations « VERS1COL F » ou « VERSICOL K » par la société ALLIED COLLOID, « UTRAHOLD 8 » par la société CIBA-GEIGY, les acides polyacryliques de type SYNTHALEN K,
- les copolymères d'acide acrylique et d'acrylamide vendus sous la forme de leur sel de sodium sous les dénominations « RETEN » par la société HERCULES, le polyméthacrylate de sodium vendu sous la dénomination « DARVAN N°7 » par la société VANDERBILT, les sels de sodium d'acides polyhydroxycarboxyliques vendus sous la dénomination « HYDAGEN F » par la société HENKEL,
- les copolymères acides polyacryliques/acrylates d'alkyle de type PEMULEN,
- l'AMPS® (Acide polyacrylamidométhyl propane sulfonique neutralisé partiellement à l'ammoniaque et hautement réticulé) commercialisé par la société CLARIANT,
- les copolymères AMPS®lacrylamide de type SEPIGEL ou SIMULGEL commercialisés par la société SEPPIC, et
- les copolymères AMPS®/méthacrylates d'alkyle polyoxyéthylénés (réticulés ou non) et leurs mélanges.

Comme autres exemples de polymères gélifiants hydrosolubles, on peut citer :

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polyuréthanes associatifs tels que le polymère C16-OE120-C16 de la société SERVO DELDEN (commercialisé sous le nom SER AD FX1100, molécule à fonction uréthane et poids moléculaire moyen en poids de 1300), OE étant un motif oxyéthyléné, le Rhéolate 205 à fonction urée vendu par la société RHBOX ou encore le Rhéolate 208 ou 204 (ces polymères étant vendus sous forme pure) ou le DW 1206B de chez RHOM & HAAS à chaîne alkyle en $C_{20}$ et à liaison uréthane, vendu à 20 % en matière sèche dans l'eau. On peut aussi utiliser des solutions ou dispersions de ces polyuréthanes associatifs notamment dans l'eau ou en milieu hydroalcoolique. A titre d'exemples de tels polymères, on peut citer le SER AD fx1010, le SER AD FX1035 et le SER AD 1070 de la société SERVO

DELDEN, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS,

- les polymères d'origine naturelle, éventuellement modifiés, tels que :
- les gommes arabiques, la gomme de guar, les dérivés du xanthane, la gomme de karaya ;
- les alginate et les carraghénanes ;
- les glycoaminoglycanes, l'acide hyaluronique et ses dérivée
- la résine shellac, la gomme de sandaraque, les dammars, les élémis, les copals ;
- l'acide désoxyribonucléique ;
- les muccopolysaccharides tels que l'acide hyaluronique, les chondroïtines sulfate, et leurs mélanges.

Certains des polymères filmogènes hydrosolubles cités plus haut peuvent également jouer le rôle de gélifiant hydrosoluble.

Les gélifiants hydrophiles peuvent être présents dans les compositions selon l'invention en une teneur allant de 0,05 à 40 % en poids par rapport au poids total de la composition, de préférence de 0,1 à 20 % et mieux de 0,5 à 15% en poids.

## Charges

La composition selon l'invention peut également comprendre au moins une charge. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, la silice traitée en surface par un agent hydrophobe, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société Dow Corning) et les microbilles de résine de silicone (Tospearls®de Toshiba, par exemple), les particules de polyorganosiloxanes élastomères, le carbonate de calcium précipité, le carbonate et l'hydra-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc de magnésium ou de lithium, le laurate de zinc, le myristate de magnésium.

On peut également utiliser un composé susceptible de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et Expancel®007WU par la Société AKZO NOBEL.

Les charges peuvent représenter de 0,1 à 25%, en particulier de 0,2 à 20 % en poids par rapport au poids total de la composition.

## Fibres

Les compositions conformes à l'invention peuvent également comprendre au moins une fibre, permettant notamment dans le cas de la mise en ouvre d'une composition de l'invention sous forme de mascara, d'obtenir une amélioration de l'effet allongeant.

Par « fibre », il faut comprendre un objet de longueur L et de diamètre D tel que L soit supérieur à D, et de préférence, très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 μm à 10 mm, de préférence de 0,1 mm à 5 mm et mieux de 0,3 mm à 3 mm. Leur section peut être comprise dans un cercle de diamètre allant de 2 nm à 500 μm, de préférence allant de 100 nm à 100 μm et mieux de 1 μm à 50 μm. Le poids ou titre des fibres est souvent donné en denier ou décitex et représente le poids en gramme pour 9 km de fil. De préférence, les fibres selon l'invention ont un titre choisi

dans la gamme allant de 0,01 à 10 deniers, de préférence de 0,1 à 2 deniers et mieux de 0,3 à 0,7 deniers.

Les fibres utilisables dans les compositions selon l'invention peuvent être choisies parmi les fibres rigides ou non rigides, elles peuvent être d'origine synthétique ou naturelle, minérale ou organique.

Par ailleurs, les fibres peuvent être traitées ou non en surface, enrobées ou non, colorées ou non colorées.

A titre de fibres utilisables dans les compositions selon l'invention, on peut citer les fibres non rigides telles que les fibres de polyamide (Nylon®) ou les fibres rigides telles que les fibres de polyimide-amide comme celles vendues sous les dénominations KERMEL® KERMEL TECH® par la société RHODIA ou de poly-(p-phénylène-téréphtalamide) (ou d'aramide) notamment vendues sous la dénomination Kevlar® par la société DUPONT DE NEMOURS.

Les fibres peuvent êtres présentes en une teneur allant de 0,01 % à 10 % en poids, par rapport au poids total de la composition, en particulier de 0,1 % à 5 % en poids, et plus particulièrement de 0,3 % à 3 % en poids.

Les compositions selon l'invention peuvent en outre comprendre tout actif cosmétique tel que les actifs choisis parmi les antioxydants, les conservateurs, les parfums, les actifs bactéricides ou anti-transpirants, les neutralisants, les émollients, les hydratants, les épaississants, les oligo-éléments, les adoucissants, les séquestrants, les agents alcalinisants ou acidifiants, les actifs hydrophiles ou lipophiles, les agents de coalescence, les plastifiants, les vitamines, des filtres en particulier solaires, et leurs mélanges.

Bien entendu, l'homme du métier veillera choisir les éventuels composés complémentaires, et/ou leur quantité, de manière telle que les propriétés avantageuses de la composition selon l'invention ne soient pas, ou substantiellement pas, altérées par l'adjonction envisagée.

## CONDITIONNEMENT

La composition selon l'invention peut être conditionnée dans un récipient délimitant au moins un compartiment qui comprend ladite composition, ledit récipient étant fermé par un élément de fermeture.

### Elément de fermeture

L'élément de fermeture peut être sous forme d'un bouchon amovible, d'un couvercle, d'un opercule, d'une bande déchirable, ou d'une capsule, notamment du type comportant un corps fixé au récipient et une casquette articulée au corps. Il peut être également sous forme d'un élément assurant la fermeture sélective du récipient, notamment une pompe, une valve, ou un clapet.

### Récipient

Le récipient peut être sous toute forme adéquate. Il peut être notamment sous forme d'un flacon, d'un tube, d'un pot, d'un étui, d'une boîte, d'un sachet ou d'un boîtier.

Le récipient peut être associé à un applicateur tel que détaillé ci-après, notamment sous forme d'une brosse.

Le produit peut être contenu directement dans le récipient, ou indirectement. A titre d'exemple, le produit peut être disposé sur un support imprégné, notamment sous forme d'une lingette ou d'un tampon, et disposé (à l'unité ou plusieurs) dans une boîte ou dans un sachet. Un tel support incorporant le produit est décrit par exemple dans la demande WO 01/03538.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par « encliquetage » on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient peut être à parois rigides ou à parois déformables, notamment sous forme d'un tube ou d'un flacon tube.

Le récipient peut comprendre des moyens destinés à provoquer ou faciliter la distribution de la composition. A titre d'exemple, le récipient peut être à parois déformables de manière à provoquer la sortie de la composition en réponse à une surpression à l'intérieur du récipient, laquelle surpression est provoquée par écrasement élastique (ou non élastique) des parois du récipient.

Le récipient peut être équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

Applicateur

L'applicateur peut prendre différentes formes. Il peut prendre notamment la forme d'une brosse comportant un arrangement de poils maintenus par un fil torsadé. Une telle brosse torsadée est décrite notamment dans le brevet US 4,887,622.

Il peut être également sous forme d'un peigne comportant une pluralité d'éléments d'application, obtenus notamment de moulage. De tels peignes sont décrits par exemple dans le brevet FR 2 796 529.

L'applicateur peut être sous forme d'un pinceau, tel que décrit par exemple dans le brevet FR 2 722 380.

L'applicateur peut être sous forme d'un bloc de mousse ou d'élastomère. L'applicateur peut être libre (éponge) ou solidaire d'une tige portée par l'élément de fermeture, tel que décrit par exemple dans le brevet US 5,492,426. L'applicateur peut être solidaire du récipient, tel que décrit par exemple dans le brevet FR 2 761 959.

La composition selon la présente invention est particulièrement avantageuse lorsqu'elle est utilisée avec un applicateur de type brosse comportant un arrangement de poils maintenus par un fil torsadé ou de type injecté, à savoir présentant une âme et des dents d'un seul tenant. En effet, ainsi qu'exposé ci-dessus, des applicateurs de ce type, et plus particulièrement de tels applicateurs ayant la propriété d'être souples, peuvent être adaptés pour l'application des compositions selon la présente invention, au cours du temps, sans l'observation des inconvénients rapportés plus haut.

Dans le cas des brosses, la souplesse résulte de différents paramètres pouvant être liés à la nature des poils, à leur section, à leur diamètre, à leur longueur et à leur densité, entre autre.

Dans le cadre de la présente invention, les éléments d'application et plus particulièrement les poils « souples », sont ceux qui présentent une résistance à la flexion limitée, tandis que les éléments d'application « durs » sont définis comme étant ceux présentant unie résistance à la flexion substantiellement plus importante.

A titre illustratif et toutes choses étant égales par ailleurs, un élément d'application court est plus dur qu'un élément d'application long et un élément d'application épais est plus dur qu'un élément d'application plus fin. De plus, les éléments d'application creux sont plus souples que les éléments d'application pleins. D'une manière générale, il existe pour les éléments d'application un diamètre au dessous duquel on les considère comme souples et au dessus duquel on les considère comme durs.

Par exemple, dans le cas de poils formés de fibres de nylon ou de polyester, les poils relativement souples ont un diamètre inférieur à 10 centièmes de millimètre, tandis que les poils relativement rigides ont un diamètre supérieur à 10 centièmes de millimètre et généralement inférieur à 30 centièmes de millimètre.

Par exemple, les poils ou dents peuvent être réalisés dans des matériaux plus ou moins souples. La dureté de ces matériaux peut être comparée par les valeurs de dureté shore. Les poils peuvent être naturels ou synthétiques. Ils peuvent être réalisés par extrusion d'une matière plastique, telle que PE, PA, notamment PA6., PA6/6, PA6/10 ou PA6/12, HYTEL®, PEBAX®, silicone, PU, cette liste n'étant pas limitative. De tels éléments d'application peuvent par exemple présenter une dureté comprise entre 20 Shore A et 40 Shore D.

On peut utiliser des poils de section transversale circulaire ou autre que circulaire. On peut par exemple utiliser des poils de section circulaire de diamètre compris entre 50 et 300 centièmes de millimètre.

Selon un mode de réalisation particulier, une composition selon l'invention peut être une composition destinée à être appliquée sur les cils, encore appelée « mascara ». Il peut s'agir d'une composition de maquillage, d'une composition cosmétique de revêtement de base appelée également « base-coat », d'une composition à appliquer sur une composition cosmétique de revêtement de base, dite encore « top-coat ». Le mascara est plus particulièrement destiné aux cils d'être humains, mais également aux faux-cils.

Les compositions selon l'invention peuvent être fabriquées par les procédés connus, généralement utilisés dans le domaine cosmétique.

L'invention est illustrée plus en détails dans les exemples suivants qui sont présentés à titre illustratif et non limitatif de l'invention.

## EXEMPLES

Dans les exemples, les pourcentages en poids sont exprimés relativement au poids total de la composition.

Exemples 1 et 2 : Mascaras avec deux alcools gras différents

| | Exemple 1 | Exemple 2 |
|---|---|---|
| Ingrédients | % en poids | % en poids |
| cire d'abeille (1) | 4,4 | 4,4 |
| cire de carnauba(2) | 3,5 | 3,5 |
| cire de paraffine (3) | 13,9 | 13,9 |
| alcool cétylique. | 4 | 0 |
| alcool béhénique, | 0 | 4 |
| oxyde de fer noir | 7,14 | 7,14 |
| gomme arabique | 0,63 | 0,63 |
| cétyl phosphate de potassium (4) | 7 | 7 |
| hydroxyéthyl cellulose (5) | 0,75 | 0,75 |
| copolymère acrylate en poids de matière première(6) | 5 | 5 |
| conservateurs & actifs | 4,36 | 4,3G |
| eau déionisée | qs | Qs |
| TOTAL | 100 | 100 |

(1) WHITE BEESWAX SP 453P commercialisé par STRAHL & PITSCH
(2) CARNAUBA WAX SP 63 commercialisé par STRAHL & PITSCH
(3) CERAFINE 56/58 PASTILLES commercialisé par BAERLOCHER
(4) AMPHISOL K commercialisé par GIVAUDAN
(5) CELLOSIZE QP 4400 H commercialisé par AMERCHOL (DOW CHEMICAL)
(6) DAITOSOL 5000 AD commercialisé par DAITO KASEI KOGYO)

Après fabrication, ces mascaras sont placés en condition de vieillissement accéléré à 45°C. La texturométrie est mesurée conformément au protocole décrit plus haut.

On observe que la formulation comprenant de l'alcool béhénique présente une valeur de texturométrie supérieure à 50 g, qui lui assure la propriété d'être bien chargeante. Sa texturométrie n'a pas besoin d'être abaissée en vue de compenser l'augmentation de la texturométrie au cours du temps.

Par ailleurs, l'évolution relative de pénétrométrie est la plus faible dans la formule comprenant l'alcool béhénique.

| | Exemple 1 alcool cétylique | Exemple 2 alcool béhénique |
|---|---|---|
| Pénétrométrie To (g) | 38 | 75 |
| Pénétrométrie après 60j à 45°C (g) | 102 | 78 |
| Evolution relative | + 268% | + 4% |

La formule de l'exemple 2 épaississant relativement moins que la formule de l'exemple 1, peut être utilisée avec une brosse de mascara plus douce que la formule de l'exemple 1. Cette brosse peut être par exemple de diamètre 9 mm avec 340 poils en élastomère polyester de diamètre 13/100. Après 60 jours en vieillissement accéléré à 45°C, la qualité du maquillage obtenue par mise en ouvre de la formule de l'exemple 2 est toujours satisfaisante avec la brosse décrite ci-dessus. En particulier, le phénomène de « sapin » ou couchage des poils après viellissement de 2 mois à 45 °C n'est pas visible lorsque cette brosse est immergée dans l'exemple 2 (alcool béhénique.). Par contre, le phénomène de « sapin » est vu lorsque cette même brosse est immergée dans l'exemple 1 (alcool cétylique) après 2 mois à 45 °C.

Exemples 3 et 4 : Mascaras avec deux alcools gras différents

| | Exemple 3 | Exemple 4 |
|---|---|---|
| Ingrédients | % en poids | % en poids |
| cire d'abeille (1) | 4,4 | 4,4 |
| cire de carnauba (2) | 3,5 | 3,5 |
| cire de paraffine (3) | 13,9 | 13,9 |
| alcool cétylique | 2 | 0 |
| alcool béhénique, | 0 | 2 |
| oxyde de fer noir | 7,14 | 7,14 |
| Steareth-2 | 2,1 | 2,1 |
| gomme arabique | 0,63 | 0,63 |
| cétyl phosphate de potassium (4) | 7 | 7 |
| hydroxyéthyl cellulose (5) | 0,75 | 0,75 |
| copolymère acrylate (6) | 5 | 5 |
| conservateurs & actifs | 4,36 | 4,36 |
| eau déionisée | qs | Qs |
| TOTAL | 100 | 100 |
| (1) WHITE BEESWAX SP 453P commercialisé par STRAHL & PITSCH<br>(2) CARNAUBA WAX SP 63 commercialisé par STRAHL & PITSCH<br>(3)CERAFINE 56/58 PASTILLES commercialisé par BAERLOCHER<br>(4) AMPHISOL K commercialisé par GIVAUDAN<br>(5) CELLOSIZE QP 4400 H commercialisé par AMERCHOL (DOW CHEMICAL)<br>(6) DAITOSOL 5000 AD commercialisé par DAITO KASEI KOGYO) | | |

Après fabrication, ces mascaras sont placés en condition de vieillissement accéléré à 45 °C. La texturométrie est mesurée conformément au protocole décrit plus haut.

On observe que l'évolution relative de Pénétrométrie est la plus faible dans la formule comprenant l'alcool béhénique.

| | Exemple 3 alcool cétylique | Exemple 4 alcool béhénique |
|---|---|---|
| Pénétrométrie To (g) | 45 | 117 |
| Pénétrométrie après 60j à 45 °C (g) | 116 | 116 |
| Evolution relative | + 258 % | -1 % |

La formulation de l'exempte 4 présente donc une meilleure stabilité relative de texture que la formule de référence de l'exemple 3 (comparatif).

## Revendications

1. Composition cosmétique pour le maquillage et/ou le soin des fibres kératiniques comprenant au moins un système émulsionnant exempt de stéarate de triéthanolamine, **caractérisée en ce qu'**elle contient au moins un pigment et de l'alcool béhénique, et **en ce que** la valeur de texture mesurée par texturométrie, selon la méthode de mesure de la texture indiquée dans la présente demande, à compter de la préparation de ladite composition, à savoir à 24

heures après la fabrication de la composition, est supérieure à 20 g à température ambiante,
ledit alcool hellénique étant présent dans une teneur supérieure ou égale à 1 % en poids par rapport au poids total de la composition,
ledit système émulsionnant comprenant au moins un agent tensioactif choisi parmi :

i) un alkyl phosphate de métal alcalin ou oxyde de phosphine de formule $(R\text{-}O)_n\text{-}P\text{=}O\text{-}(O\,M)_m$ avec R représentant un groupe alkyle, linéaire ou ramifié, en $C_8$-$C_{22}$ tel que le cétyle, n étant égal à 1, 2 ou 3 et m étant égal à 0, 1 ou 2, avec m + n étant égal à 3 et M représentant un atome d'hydrogène ou un métal alcalin ou alcalino terreux, préférentiellement n = 1 et m = 2, et M est un métal alcalin, tel que le sodium ou le potassium,
ii) un alcool polyéthoxylé de formule $R'\text{-}(OCH_2CH_2)p\text{-}OH$ avec R' représentant un alkyle linéaire ou ramifié en $C_1$-$C_{30}$ et particulièrement représente $CH_3\text{-}(CH_2)_{17}\text{-}$ et p représentant un entier compris inclusivement entre 1 et 30, préférentiellement entre 2 et 20 ; tels que le stéareth-20 et le stéareth-2,
iii) un sel d'acide glutamique de formule $R\text{-}CONH\text{-}C(COOM)\text{-}C_2H_4\text{-}COO\text{-}M'$ avec R représentant un groupe alkyle, linéaire ou ramifié en $C_8$-$C_{22}$ tel que le stéaryle et M' représentant un métal alcalin ou alcalino terreux, et
iv) un alkyl glucoside obtenu par condensation de glucose et d'alcools gras linéaires ou ramifiés en $C_8$-$C_{22}$ tel qu'un mélange cétylique et stéarique nommé cétylstéaryle.

**2.** Composition pour le maquillage et/ou le soin des fibres kératiniques selon la revendication 1, ledit système émulsionnant comprenant au moins un agent tensioactif selon le point (i) et/ou au moins un agent tensioactif selon le point (iii), ainsi qu'éventuellement au moins un agent tensioactif selon le point (ii) et/ou au moins un agent tensioactif selon le point (iv) et/ou au moins un alcool gras comprenant de 10 à 26 atomes de carbone, de préférence de 10 à 24 atomes de carbone, et plus préférentiellement de 12 à 21 atomes de carbone.

**3.** Composition cosmétique pour le maquillage et/ou le soin des fibres kératiniques selon la revendication 1 ou 2, **caractérisée en ce que** la valeur de texture mesurée par texturométrie à compter de la préparation de ladite composition, à savoir 24 heures après la fabrication de la composition, est supérieure à 30 g à température ambiante, voire à 60 g, ou encore à 70 g.

**4.** Composition cosmétique pour le maquillage et/ou le soin des fibres kératiniques selon l'une quelconque des revendications précédentes, **caractérisée en ce que** la valeur de texture mesurée par texturométrie à l'issue d'une période de 60 jours à 45°C est inférieure ou égale à 100 g, voire à 90 g.

**5.** Composition cosmétique pour le maquillage et/ou le soin des fibres kératiniques selon l'une quelconque des revendications précédentes, **caractérisée en ce que** la variation de texture mesurée par texturométrie, dans une période de 60 jours à 45°C à compter de la préparation de ladite composition, à savoir à 24 heures après la fabrication de la composition, est inférieure à 100 %, la variation de texture étant définie par :

$$\frac{T_{60\,j} - T_0}{T_0} \times 100$$

où T60j est la mesure de texturométrie à 60 jours, et
$T_0$ est la mesure de texturométrie 24 heures après fabrication de la composition.

**6.** Composition cosmétique selon la revendication précédente, **caractérisée en ce que** la variation de texture mesurée par texturométrie dans une période de 60 jours à 45 °C à compter de la préparation de ladite composition est inférieure à 70 %, voire à 60 %, voire à 50 %, par exemple à 20 %, 10 %, ou encore 5 % et plus particulièrement dans une teneur variant de 0,3 % à 20 % en poids, notamment de 0,5 % à 10 % en poids et par exemple de 0,7 % à 7 %, voire de 1 % à 6 % en poids par rapport au poids total de la composition.

**7.** Composition cosmétique selon l'une quelconque des revendications précédentes, comprenant une phase aqueuse continue.

**8.** Composition cosmétique selon l'une quelconque des revendications précédentes, **caractérisée en ce que** l'alcool béhénique est présent dans une teneur supérieure ou égale à 2 % en poids, par rapport au poids total de la

composition.

**9.** Composition cosmétique selon l'une quelconque des revendications précédentes, caractérisée en ce le système émulsionnant comprend au moins un éther oxyéthyléné et/ou oxypropyléné pouvant comporter de 1 à 150 groupes oxyéthylénés et/ou oxypropylénés, d'alcool en $C_8$-$C_{24}$, et de préférence en $C_{12}$-$C_{18}$, tel que l'éther oxyéthyléné de l'alcool stéarylique à 2 groupes oxyéthylénés, et un alkyl phosphate de métal alcalin ou oxyde de phosphine de formule $(R\text{-}0)_n\text{-}P\text{=}0\text{-}(0^{-}M)_m$ avec R représentant un groupe alkyle, linéaire ou ramifié, en $C_8$-$C_{22}$ tel que le cétyle, n étant égal à 1, 2 ou 3 et m étant égal à 0, 1 ou 2, avec m + n étant égal à 3 et M représentant un atome d'hydrogène ou un métal alcalin ou alcaline terreux, préférentiellement n = 1 et m = 2, et M est un métal alcalin, tel que le sodium ou le potassium à titre d'agents tensioactifs.

**10.** Composition cosmétique selon l'une quelconque des revendications précédentes, **caractérisée en ce que** le système émulsionnant comprend au moins un agent tensioactif choisi parmi le cétyl phosphate de potassium, le stéareth-2, le stéareth-20 et leur mélange.

**11.** Composition cosmétique selon l'une quelconque des revendications 1 à 8, **caractérisée en ce que** le système émulsionnant comprend au moins un agent tensioactif choisi parmi le glutamate de stéaroyle de sodium, le glucoside de cétylstearyle et leur mélange.

**12.** Composition cosmétique selon l'une quelconque des revendications précédentes, **caractérisée en ce que** le système émulsionnant comprend un agent tensioactif de HLB supérieur à 8 en association avec un agent tensioactif de HLB inférieur à 8.

**13.** Composition cosmétique selon l'une quelconque des revendications précédentes, **caractérisée en ce qu'**elle comprend en outre au moins un agent structurant lipophile tel que les cires, les corps gras pâteux et leurs mélanges.

**14.** Ensemble de conditionnement et d'application comprenant un récipient renfermant une composition selon l'une quelconque des revendications 1 à 13 et un applicateur configuré pour appliquer ladite composition sur une matière kératinique, et en particulier sur les fibres kératiniques, telles que les cils ou sourcils, ledit applicateur comprenant des éléments d'application, tels que des poils ou des dents, présentant une dureté comprise entre 20 Shore A et 40 Shore D.

**15.** Procédé de revêtement des fibres kératiniques, telles que les cils ou les sourcils comprenant une étape d'application d'une composition selon l'une quelconque des revendications 1 à 1.3 sur lesdites fibres kératiniques.

Europäisches Patentamt
European Patent Office
Office européen des brevets

# RAPPORT DE RECHERCHE EUROPEENNE

Numéro de la demande
EP 10 19 4784

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | DATABASE WPI<br>Week 200426<br>Thomson Scientific, London, GB;<br>AN 2004-273106<br>XP002633102,<br>& JP 2004 010496 A (NIPPON SHOKUZAI KOGYO KENKYUSHO) 15 janvier 2004 (2004-01-15)<br>* abrégé * | 1-15 | INV.<br>A61K8/34<br>A61Q1/10<br>A61K8/55<br>A61K8/92<br>A45D34/04<br>A45D40/26 |
| Y | EP 1 920 759 A1 (OREAL [FR])<br>14 mai 2008 (2008-05-14)<br>* alinéas [0001] - [0010], [0018] - [0030], [0042]; exemples 6-8 * | 1-15 | |
| Y | FR 2 908 302 A1 (OREAL [FR])<br>16 mai 2008 (2008-05-16)<br>* page 1, ligne 26 - page 2, ligne 6 *<br>* page 4, ligne 15 - page 5, ligne 32 *<br>* page 7, ligne 10-19 *<br>* page 9, ligne 15-17; exemples * | 1-15 | DOMAINES TECHNIQUES RECHERCHES (IPC) |
| Y | WO 2008/066243 A1 (AMOREPACIFIC CORP [KR]; PARK SE JUN [KR]; KWON SUN SANG [KR]; ROH YOUN) 5 juin 2008 (2008-06-05)<br>* exemple 1 * | 1-15 | A61K<br>A61Q<br>A45D |
| Y | WO 2006/116731 A2 (PROCTER & GAMBLE [US]; OSBORNE ROSEMARIE [US])<br>2 novembre 2006 (2006-11-02)<br>* exemples 4, 5, 21, 22 *<br>* page 7, ligne 14 *<br>-/-- | 1-15 | |

Le présent rapport a été établi pour toutes les revendications

1

| Lieu de la recherche | Date d'achèvement de la recherche | Examinateur |
|---|---|---|
| Munich | 15 avril 2011 | Perrone Dunet, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

Europäisches Patentamt
European Patent Office
Office européen des brevets

# RAPPORT DE RECHERCHE EUROPEENNE

Numéro de la demande
EP 10 19 4784

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | EP 0 509 852 A1 (OREAL [FR]) 21 octobre 1992 (1992-10-21)<br>* figure 1 *<br>* colonne 1, ligne 1-8, 30-51 *<br>* colonne 3, ligne 23-26 *<br>* colonne 4, ligne 45-46 *<br>* revendication 1 *<br>----- | 14 | |
| A | FR 2 881 642 A1 (OREAL [FR]) 11 août 2006 (2006-08-11)<br>* page 1, ligne 10, 27-31 *<br>* page 2, ligne 1-24 *<br>* page 5, ligne 9-11 *<br>* page 15, ligne 31 - page 16, ligne 35 *<br>* exemple 2 *<br>----- | 1-15 | |
| A | EP 1 733 711 A1 (KAO CORP [JP]) 20 décembre 2006 (2006-12-20)<br>* exemples 6-14 *<br>* examples comparatifs 3-6 *<br>----- | 1-15 | DOMAINES TECHNIQUES RECHERCHES (IPC) |
| A | WO 2006/082102 A2 (SCHWAN STABILO COSMETICS GMBH [DE]; SWISTOWSKI AZRA [DE]; ZECH CHRISTI) 10 août 2006 (2006-08-10)<br>* page 5, ligne 15-18 *<br>* exemples 1, 2 *<br>----- | 1-15 | |

1

Le présent rapport a été établi pour toutes les revendications

| Lieu de la recherche | Date d'achèvement de la recherche | Examinateur |
|---|---|---|
| Munich | 15 avril 2011 | Perrone Dunet, S |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 10 19 4784

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-04-2011

| Document brevet cité au rapport de recherche | Date de publication | Membre(s) de la famille de brevet(s) | Date de publication |
|---|---|---|---|
| JP 2004010496 A | 15-01-2004 | AUCUN | |
| EP 1920759 A1 | 14-05-2008 | CN 101176701 A | 14-05-2008 |
| | | FR 2908307 A1 | 16-05-2008 |
| | | JP 2008120817 A | 29-05-2008 |
| | | US 2009016982 A1 | 15-01-2009 |
| FR 2908302 A1 | 16-05-2008 | AUCUN | |
| WO 2008066243 A1 | 05-06-2008 | KR 100828461 B1 | 13-05-2008 |
| WO 2006116731 A2 | 02-11-2006 | AU 2006239234 A1 | 02-11-2006 |
| | | CA 2604962 A1 | 02-11-2006 |
| | | CN 101166512 A | 23-04-2008 |
| | | EP 1879549 A2 | 23-01-2008 |
| | | JP 2008538769 T | 06-11-2008 |
| | | KR 20070116134 A | 06-12-2007 |
| | | US 2007020220 A1 | 25-01-2007 |
| EP 0509852 A1 | 21-10-1992 | CA 2064418 A1 | 17-10-1992 |
| | | DE 69225350 D1 | 10-06-1998 |
| | | DE 69225350 T2 | 28-01-1999 |
| | | ES 2115651 T3 | 01-07-1998 |
| | | FR 2675355 A1 | 23-10-1992 |
| | | JP 5123217 A | 21-05-1993 |
| | | US 5238011 A | 24-08-1993 |
| FR 2881642 A1 | 11-08-2006 | AUCUN | |
| EP 1733711 A1 | 20-12-2006 | WO 2005089701 A1 | 29-09-2005 |
| | | KR 20060130656 A | 19-12-2006 |
| | | US 2007202066 A1 | 30-08-2007 |
| WO 2006082102 A2 | 10-08-2006 | CA 2524079 A1 | 04-08-2006 |
| | | DE 102005005486 A1 | 17-08-2006 |
| | | EP 1843821 A2 | 17-10-2007 |
| | | JP 2008528655 T | 31-07-2008 |
| | | US 2006177391 A1 | 10-08-2006 |
| | | US 2007122368 A1 | 31-05-2007 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2792190 A
- WO 061013413 A
- WO 20071068371 A
- WO 2008155059 A
- WO 200815505 A
- US 2004175338 A
- US 6491927 B
- EP 847752 A
- FR 2679771
- EP 1184426 A
- FR 2232303 A
- US 5162410 A
- WO 2004073626 A
- US 5874069 A
- US 5919441 A
- US 6051216 A
- US 5981680 A
- EP 1411069 A
- WO 04028488 A
- WO 04055081 A
- WO 0103538 A
- FR 2792618
- US 4887622 A
- FR 2796529
- FR 2722380
- US 5492426 A
- FR 2761959


### Littérature non-brevet citée dans la description


- **GRIFFIN.** *J. Soc. Cosm. Chem.,* 1954, vol. 5, 249-256
- Encyclopedia o*f* Chemical Technology, KIRK-OTHMER. WILEY, 1979, vol. 22, 333-432
- Microemulsions Theory and Practice. Academic Press, 1977, 21-32